# EUROPEAN PATENT SPECIFICATION

(11) **EP 2 563 398 B1**
(45) Date of publication and mention of the grant of the patent: **19.04.2017**
(21) Application number: 11777722.7
(22) Date of filing: 21.01.2011
(51) Int. Cl.: A61K 39/00, A61K 38/27, A61K 39/395, C07K 16/26, C07K 16/28

(54) **GROWTH HORMONE RECEPTOR DEFICIENCY CAUSES A MAJOR REDUCTION IN PRO-AGING SIGNALING, CANCER AND DIABETES IN HUMANS**
WACHSTUMSHORMONREZEPTORMANGEL ALS URSACHE EINER GRÖSSEREN ABNAHME VON PRO-AGING-SIGNALEN SOWIE VON KREBS UND DIABETES BEIM MENSCHEN
FORTE DIMINUTION DE LA SIGNALISATION PRO-VIEILLISSEMENT, DU CANCER ET DU DIABÈTE CHEZ L'ÊTRE HUMAIN SOUS L'EFFET D'UN DÉFICIT EN RÉCEPTEURS DE L'HORMONE DE CROISSANCE

(30) Priority: 28.04.2010 US 328812 P
(43) Date of publication of application: 06.03.2013
(73) Proprietor: University of Southern California, Los Angeles, CA 90089-2561 (US)
(72) Inventor: LONGO, Valter, D., Playa del Rey, CA 90293 (US); GUEVARA, Jaime, Los Angeles, CA 90089 (US)
(74) Representative: Lau, Sarah Jane
(86) International application number: PCT/US2011/022052
(87) International publication number: WO 2011/139391

(56) References cited:
- WO-A2-2004/022005
- WO-A2-2005/041901
- US-A1- 2004 121 407
- US-A1- 2007 185 031
- US-B2- 7 173 005
- GN AJAY THANKAMONY ET AL.: "Pegvisomant: current and potential novel therapeutic applications", EXPERT OPINION ON BIOLOGICAL THERAPY, vol. 9, no. 12, December 2009 (2009-12), pages 1553-1563, XP008164766, DOI: 10.1517/14712590903449222
- CHANGHAN LEE ET AL.: 'Reduced IGF-I differentially protects normal and cancer cells and improves chemotherapeutic index in mice' CANCER RESEARCH vol. 70, no. 4, 15 February 2010, pages 1564 - 1572, XP055178004
- STEPHAN SEITZ ET AL: 'Inhibition of estrogen receptor positive and negative breast cancer cell lines with a growth hormone-releasing hormone antagonist' ONCOLOGY REPORTS, [Online] 01 November 2008, GREECE, pages 1289 - 1294, XP55165843 DOI: 10.3892/or_00000143 Retrieved from the Internet: <URL:http://www.ncbi.nlm.nih.gov/pubmed/189 49435> [retrieved on 2015-01-29]

### SEQUENCE LISTING

<110> University of Southern California
<120> GROWTH HORMONE RECEPTOR DEFICIENCY CAUSES A MAJOR REDUCTION IN PRO-AGING SIGNALING, CANCER AND DIABETES IN HUMANS
<130> USC0109PCT
<160> 5
<170> PatentIn version 3.5
<210> 1
   <211> 42
   <212> DNA
   <213> Homo sapiens
<400> 1
   caatacaaag aggtaaatga aactaaatgg aaaatggtaa ga 42
<210> 2
   <211> 9
   <212> PRT
   <213> Homo sapiens
<400> 2
   Glu Val Asn Glu Thr Lys Trp Lys Met
   1               5
<210> 3
   <211> 20
   <212> DNA
   <213> Artificial Sequence
<220>
   <223> reverse primer for genotyping the wild type GHR gene
<400> 3
   cattgccctc aactggactt 20
<210> 4
   <211> 25
   <212> DNA
   <213> Artificial Sequence
<220>
   <223> reverse primer for genotyping the wild type GHR gene
<400> 4
   cattttccat ttagtttcat ttact 25
<210> 5
   <211> 24
   <212> DNA
   <213> Artificial Sequence
<220>
   <223> reverse primer for genotyping the mutant GHR gene
<400> 5
   cattttccat ttagtttcat ttac 24

## Description

### BACKGROUND OF THE INVENTION

### 1. Field of the Invention

The present disclosure relates to methods of reducing the deleterious effects of aging, oxidative damage and chemotherapy in a subject and preventing and/or alleviating a symptom of age related diseases.

### 2. Background Art

CHANGHAN LEE ET AL Cancer Research, vol. 70, no. 4, 15 February 2010 (2010-02-15), pages 1564-1572, demonstrates the use of an antibody directed against an IGF-I receptor in chemotherapy in a subject. It also measures the level of IGF-1 in the subject following injection of the antibody and cyclophosphamide (Cyclophosphamide is used to treat cancers and autoimmune disorders). Reduced activity of growth hormone (GH) and insulin like growth factor-I (IGF-I) signaling or of their orthologs in lower organisms, and the activation of stress resistance transcription factors and antioxidant enzymes, contribute to extended life span and protection against age-dependent damage or diseases (*1-15*). Pathways that normally regulate growth and metabolism also promote aging and genomic instability, a correspondence that is conserved in simple eukaryotes and mammals (*7, 16-18*). In yeast, life span extending mutations in genes such as *SCH9,* the homolog of mammalian S6K, protect against age-dependent genomic instability (*19, 20*). Similarly, mutations in the insulin/IGF-I like signaling (IIS) pathway increase lifespan and reduce abnormal cellular growth in worms, and mice deficient in GH and IGF-I are not only long-lived but also exhibit a delayed occurrence of age-dependent mutations and neoplastic disease (*5*, *6, 21-25*). Among the most frequently detected mutations in human cancers are those that activate the two main signaling proteins downstream of the IGF-I receptor: Ras and Akt, and those in the IGF-1 receptor itself (*26, 27*). This is in agreement with a potential role for the IGF-I signaling pathway in promoting age-dependent mutations that lead to tumorigenesis and for mutated proto-oncogenes in exacerbating the generation of mutations (*28*). It has been proposed that the growth-promoting and anti-apoptotic functions of the IGF-1 pathway underlie its putative role in cancer development and progression (*29*). However, this link is not supported by population studies in humans, which indicate only a modest association between high IGF-I concentrations and increased risk of certain cancers (*29*, *30*). GH may also promote insulin resistance. For example, age-dependent insulin resistance is reduced in GH deficient mice (*31-34*), and GH replacement therapy can exacerbate insulin resistance in GH-deficient individuals (*35*, *36*), apparently because it causes a switch from glucose metabolism to lipolysis (*37*).

Although advantageous of inducing low GH and/or IGF-I levels in a subject in treating several ailments such as acromegaly are known, the extent of the benefits of modifying GH and/or IGF-I levels in a subject requires further development. Accordingly, there is a need for additional methods for alleviating disease symptoms utilizing GH and IGF-I modification.

### SUMMARY OF THE INVENTION

Against this prior art background, the present disclosure describes in at least one embodiment a method of inhibiting development of a symptom aging in a subject. The method comprises identifying a subject that does not suffer from acromegaly of less than 70 years of age with IGF-I levels in the highest quartile of a population and then administering a therapeutically effective amount of a GH/IGF-1 Axis inhibitory composition to the subject so that IGF-I levels are reduced to the median level for that population. Typically, the levels of IGF-1 and insulin in the subject are monitored.

The present invention provides a GH/IGF-1 Axis inhibitory composition for use in a method for reducing chemotherapy side effects in a subject as set out in the claims. The method comprises identifying a subject undergoing chemotherapy and then administering a therapeutically effective amount of a GH/IGF-1 Axis inhibitory composition to the subject. Typically, chemotherapy related symptoms and the levels of IGF- 1 in the subject are monitored.

The present disclosure describes a method for alleviating a symptom of oxidative damage in a subject. The method comprises identifying a subject with an IGF-I level in the upper half of the normal age- and sex-specific levels of IGF-I compared to general population (excluding subjects diagnosed with acromegaly) and then administering a therapeutically effective amount of a GH/IGF-1 Axis inhibitory composition to the subject so that the levels fall to below the median. Typically, the levels of IGF- 1 and insulin in the subject are monitored.

In another embodiment, the present disclosure describes a method for inhibiting the development of a symptom of aging. The method comprises identifying a subject with an IGF- I level in the upper half of the normal age- and sex-specific levels of IGF-I compared to general population (for example excluding subjects diagnosed with acromegaly) and then administering a therapeutically effective amount of a GH/IGF-1 Axis inhibitory composition to the subject so that the levels fall to below the median. Typically, the levels of IGF-1 and insulin in the subject are monitored.

In another embodiment, the present disclosure describes a method for inhibiting the development of a symptom of cancer or the risk of developing cancer in a subject. The method comprises identifying a subject with an IGF-I level in the upper half of the normal age- and sex-specific levels of IGF-I compared to an average for the general population (for example excluding subjects diagnosed with acromegaly) and then administering a therapeutically effective amount of a GH/IGF-1 Axis inhibitory composition to the subject so that the levels fall to below the median. Typically, the levels of IGF-1 and insulin in the subject are monitored.

In another embodiment, the present disclosure describes a method for inhibiting development of a symptom of diabetes in a subject. The method comprises identifying a subject with an IGF-I level in the upper half of the normal age- and sex-specific levels of IGF-I compared to an average for the general population (for example, excluding subjects diagnosed with acromegaly) and then administering a therapeutically effective amount of a GH/IGF-1 Axis inhibitory composition to the subject so that the levels fall to below the median. Typically, the levels of IGF-1 and insulin in the subject are monitored.

In still another embodiment, the present disclosure describes a method for reducing oxidative damage in various eukaryotic cells. The method comprises identifying a eukaryotic cell predisposed to oxidative damage and then administering a therapeutically effective amount of a GH/IGF-1 Axis inhibitory composition to the subject.

Some of the advantages of various embodiments of the invention in humans were tested by monitoring 99 Ecuadorian subjects with mutations in the growth hormone receptor gene leading to GHRD and severe IGF-I deficiency were monitored for 22 years. This combined information was combined with surveys to identify the cause and age of death for GHRD subjects who died before this period. Surprisingly, individuals with GHRD exhibited only one non-lethal malignancy and no cases of diabetes, in contrast to the expected incidence of these diseases in their age -matched relatives. As set forth below, a potential explanation for the very low incidence of cancer comes from in vitro studies which revealed an effect for serum from GHRD subjects on both reduction of DNA breaks but increase in the apoptosis of primary human mammary epithelial cells (HMECs) exposed to hydrogen peroxide. Reduced insulin concentrations (1.4 µU/ml vs. 4.4µU/ml) and a very low homoeostasis model assessment of insulin resistance (HOMA-IR) index (0.33 vs. 0.95) in GHRD individuals is observed, indicating increased insulin sensitivity, which could explain the absence of diabetes in these subjects. Incubation of HMECs with GHRD serum also caused reduced expression of Ras, PKA and TOR, and up-regulation of SOD2, changes implicated in cellular protection and life span extension in model organisms.

### BRIEF DESCRIPTION OF THE DRAWINGS

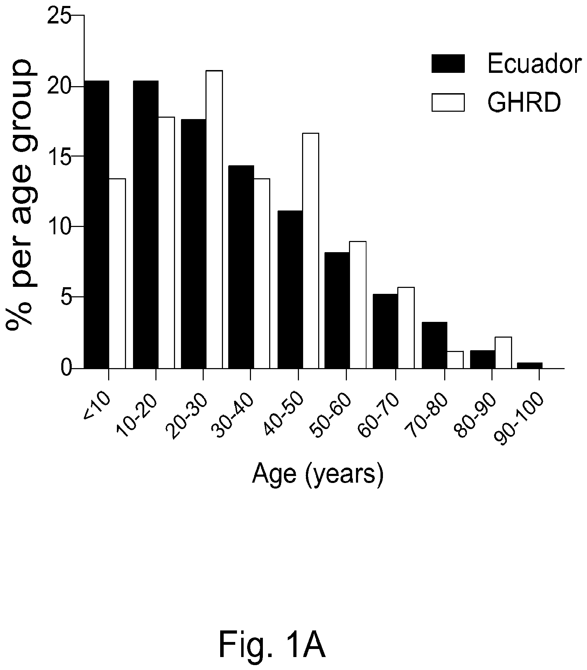

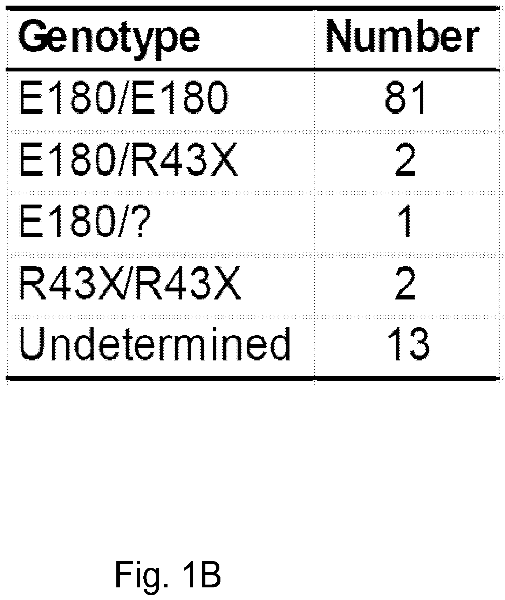

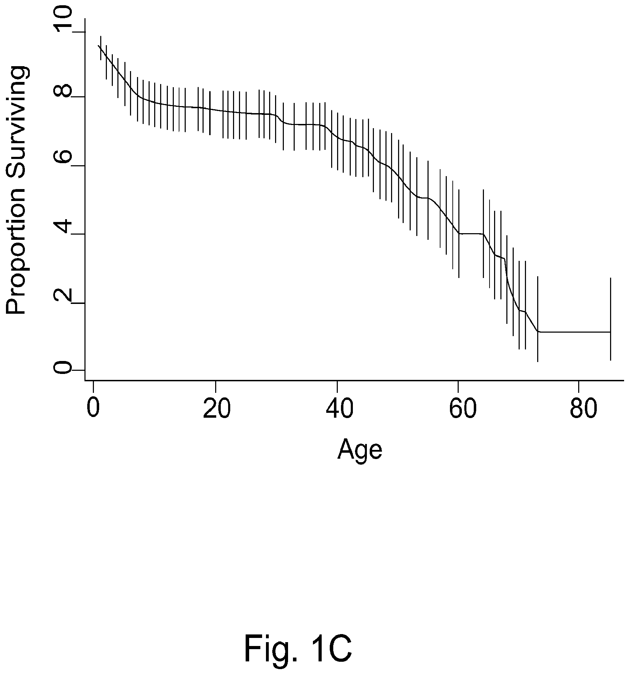

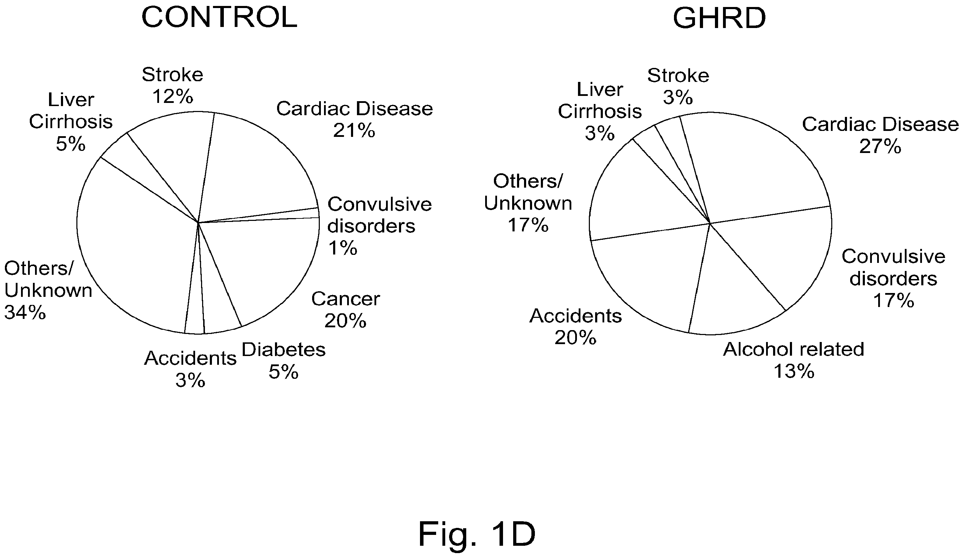

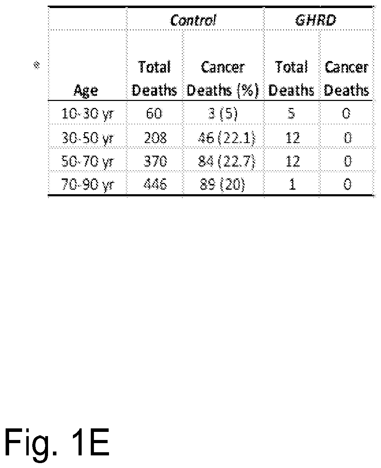

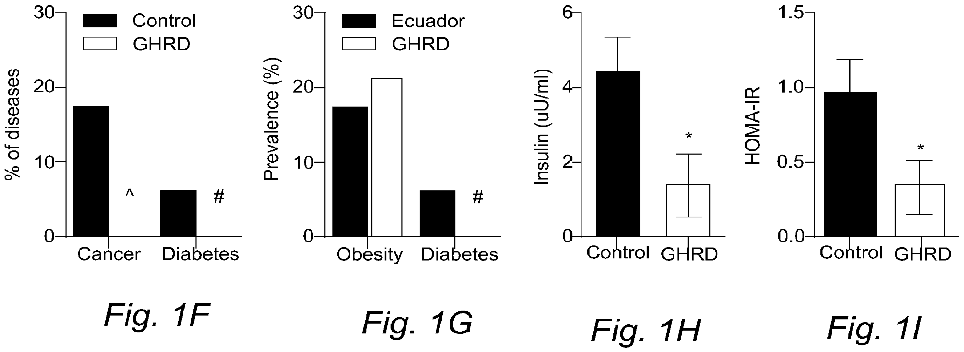

FIGURE 1 provides data for the Ecuadorian GHRD cohort used in part of the study set forth below: (A) Age distribution for 90 Ecuadorian GHRD subjects and the general Ecuadorian population. (B) Genotypes of the Ecuadorian GHRD cohort. All GHRD subjects were identified based on short stature and very low IGF-I levels. One individual with the GHRD phenotype is heterozygous for the E180 mutation. The term "undetermined" refers to subjects whose genotypes have not been confirmed. (C) Survival of the GHRD cohort. (D, E) Cause of death (D) and percentage of cancers per age group (E) in control and GHRD subjects. (F) Percentage of cancer and type II diabetes in control and GHRD subjects. Data are shown as a percentage of all diagnosed/reported diseases. ^, 1 case of cancer and #, no case of diabetes has been recorded. (G) Prevalence of obesity and type II diabetes prevalence in Ecuador and the GHRD cohort. #, no case of diabetes has been recorded. Obesity prevalence in Ecuador is based on WHO reports (*48*) while that for GHRD subjects was calculated based on BMI > 30 kg/m². Prevalence of type II diabetes in Ecuador was obtained from the study by Shaw S.E. et al (*49*). (H, I) Fasting serum insulin levels (H) and Homeostatic Model Assessment-Insulin Resistance (HOMA-IR) (I) in relatives and GHRD subjects. Data represent mean ± SEM for 13 control and 16 GHRD samples, * p<0.05.

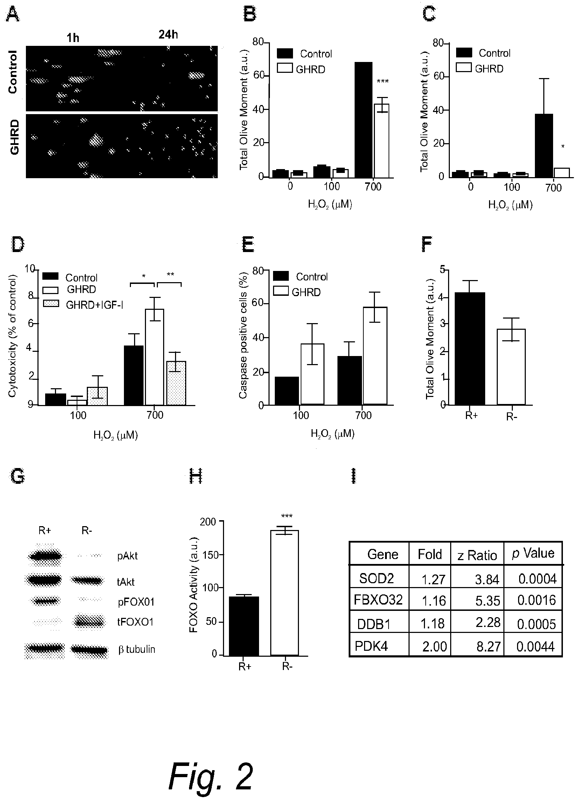

FIGURE 2 provides data showing that reduced IGF-1 signaling protects against DNA damage and favors apoptosis of damaged cells: (A) Representative micrographs of DNA damage in epithelial cells treated with H₂O₂ for 1 hour or 24 hours. (B, C) Tail olive moment to measure DNA breaks in epithelial cells treated with H₂O₂ for 1 hour (B) or 24 hours (C). Data represent mean ± SEM. At least 6 serum samples were tested per group and 100-200 cells were analyzed per sample. (D) LDH activity in epithelial cells incubated with control or GHRD serum and treated with H₂O₂ for 24 hours. Data represent mean ± SEM. 6 serum samples were tested per group in triplicates. (E) Activation of caspases in epithelial cells incubated with control or GHRD serum and treated with H₂O₂ for 6 hours. Data are calculated as percentage of untreated control and represent mean ± SEM. 6 serum samples were tested per group. (F) Tail olive moment to measure basal DNA damage in R+ (IGF-IR overexpression) or R- (IGF-IR deficient) mouse embryonic fibroblasts (MEFs). Data represent mean ± SEM. (G) Representative western blot showing phosphorylation status of Akt (Ser 473) and FoxO1 (Ser 256) in R+ and R- cells. (H) FoxO activity in R+ and R- cells transfected with a luciferase reporter plasmid. (I) List of FoxO target genes significantly upregulated in human epithelial cells incubated in GHRD serum versus control serum, *p<0.05, **p<0.01, ***p<0.0001.

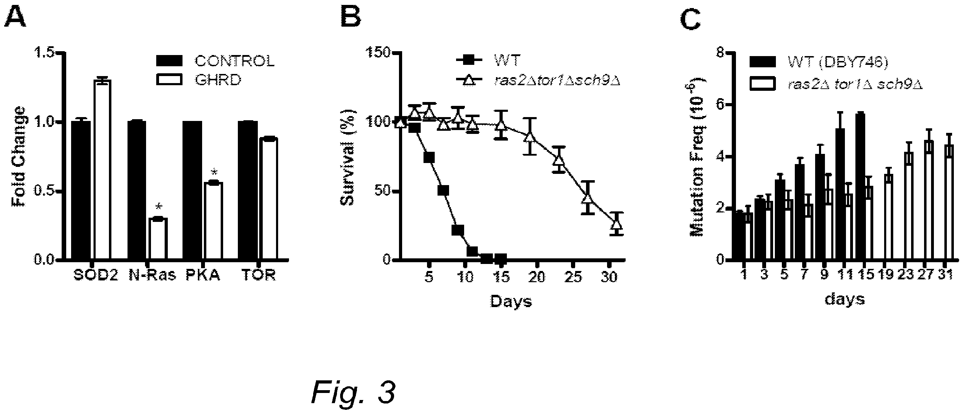

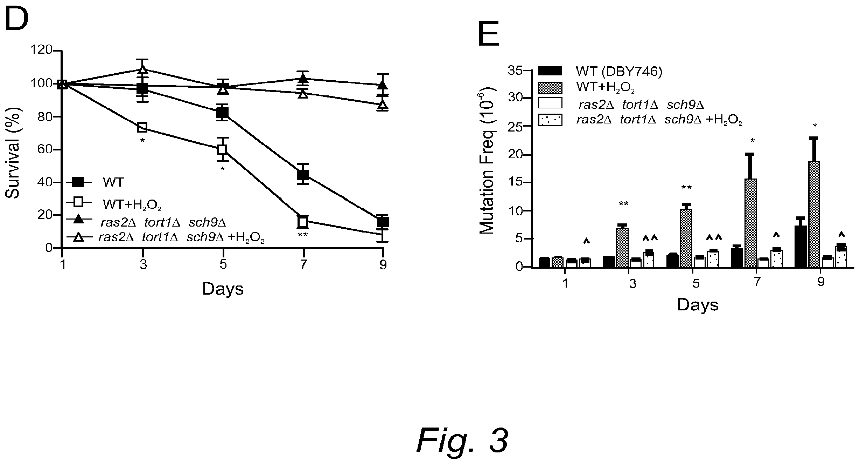

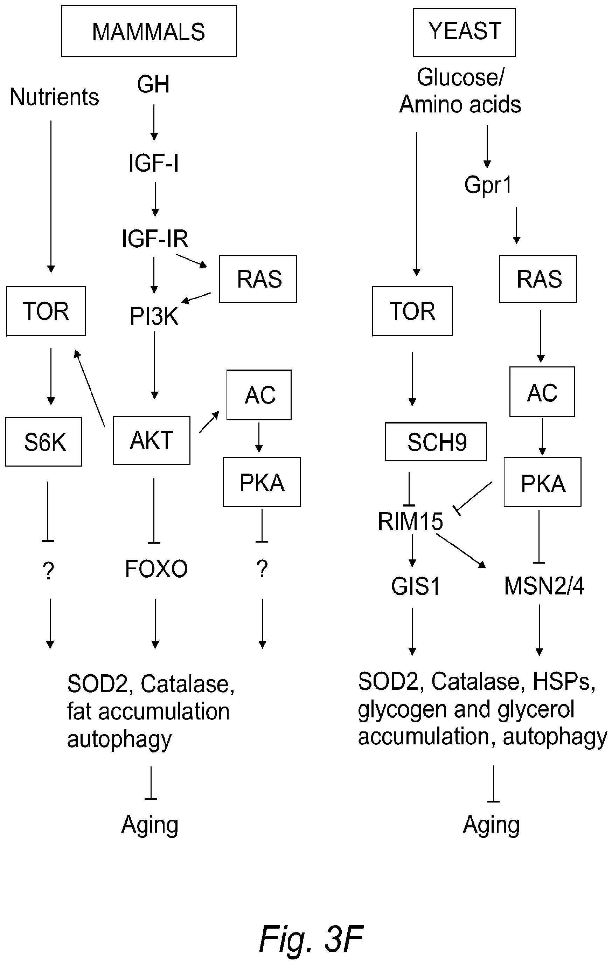

FIGURE 3 provides data showing the protective effects of reduced pro-growth signaling in yeast and mammals: (A) RT-PCR confirmation of the upregulation of SOD2 and downregulation of N-Ras, PKA and TOR in human epithelial cells incubated in GHRD serum. (B) Chronological survival of WT and *ras2*Δ*, sch9Δ, tor1Δ* yeast triple mutants. (C) Mutation frequency over time in the *CAN1* gene (measured as Can^{r} mutants/10⁶ cells). (D) Chronological survival of WT and *ras2*Δ*, sch9Δ, tor1Δ* yeast triple mutants in response to H₂O₂ induced oxidative stress. (E) Mutation frequency over time in the *CAN1* gene (measured as Can^{r} mutants/10⁶ cells) in response to H₂O₂ induced oxidative stress. Data represent mean ± SEM, n= 5. *p<0.05, **p<0.01 compared to WT untreated cells. (F) Schematic representation of conserved growth factor signaling pathways in mammals and yeast;
FIGURES 4A-E provide a questionnaire used for data collection from interviews. At least 2 family members were required to be present at the time of the interview. Only the causes of death confirmed by at least 2 relatives were included in the study. The genotype of deceased GHRD subjects was inferred based on clinical phenotype and pedigree information provided by the relatives;
FIGURE 5 provides nucleotide and amino acid sequence (SEQ ID NO: 1 and SEQ ID NO: 2) of the E180 A to G base substitution which results in an alternative splice site in the GHR gene for the Ecuadorian GHRD cohort;
FIGURE 6 provides IGF measurements in serum. IGF-I and IGF-II levels were measured in serum from 13 relatives and 16 GHRD subjects by ELISA. ****p<0.0001*;
FIGURE 7 provides percentages of different cancer related deaths in unaffected relatives of GHRD subjects;
FIGURE 8 provides fasting serum glucose levels in control and GHRD subjects;
FIGURE 9 provides LDH activity in mouse embryonic fibroblasts incubated in control or GHRD serum and treated with H₂O₂. ***p<0.001,* ****p<0.0001*;
FIGURE 10 provides a table that shows a list of genes with significant differences in expression between epithelial cells incubated in either control or GHRD serum;
FIGURE 11 shows functional clustering of genes with significant differences in expression between epithelial cells incubated in either control or GHRD serum;
FIGURE 12 provides a table that shows genes in the top four functional groups with significant differences in expression identified by microarray analysis;
FIGURE 13A provides a plot of body weight of mice immunized with human Growth Hormone (huGH) (20 male and 20 female) versus time. Lower body weight in these mice indicate that inhibitory antibodies against human GH have been generated by the mice;
FIGURE 13B provides an image that shows the anti-huGH activity in the serum from immunized mice were used to blot the immobilized human GH (slot blot) - 34 out of 40 mice immunized with huGH showed strong activity;
FIGURE 14A provides plots of the body weight of mice with short term immunization (STI) with human GH (half-filled arrowheads) after cyclophosphamide treatment (cyclophosphamide (CP) i.p., 300 mg/kg, indicated by the solid arrowhead) ( Sham means treated with buffer and no chemo);
FIGURE 14B provides a histogram of the complete blood count (CBC) 7-days after CP treatment (Data are presented as percentage of pre-chemo value);
FIGURE 15 shows the efficacy of the growth hormone receptor antagonist. Mouse L cell fibroblasts expressing GHR were serum starved for 24 hours, then treated with 5nM growth hormone (GH) for 5 min either without or without a 30 min, 50nM growth hormone antagonist (GHA) pre-treatment. The phospho stat5 signal reflects the activity of the growth hormone receptor;
FIGURE 16 provides a plot of an experiment in which human stem cells from amniotic fluid were pre-treated with an inhibitory antibody that blocks the IGF-I receptor before treatment with different doses of the chemotherapy drug cyclophosphamide at the indicated doses; and
FIGURE 17 provides a plot of the 24 hour lactate dehydrogenase (LDH) release from the treatment of primary glial cells with the IGF-I inhibitor IGFBP1 and with IGFBP1 protects them against oxidative damage.

### DETAILED DESCRIPTION OF THE PREFERRED EMBODIMENT(S)

Reference will now be made in detail to presently preferred compositions, embodiments and methods of the present invention, which constitute the best modes of practicing the invention presently known to the inventors. The Figures are not necessarily to scale. However, it is to be understood that the disclosed embodiments are merely exemplary of the invention that may be embodied in various and alternative forms. Therefore, specific details disclosed herein are not to be interpreted as limiting, but merely as a representative basis for any aspect of the invention and/or as a representative basis for teaching one skilled in the art to variously employ the present invention.

Except in the examples, or where otherwise expressly indicated, all numerical quantities in this description indicating amounts of material or conditions of reaction and/or use are to be understood as modified by the word "about" in describing the broadest scope of the invention. Practice within the numerical limits stated is generally preferred. The description of a group or class of materials as suitable or preferred for a given purpose in connection with the invention implies that mixtures of any two or more of the members of the group or class are equally suitable or preferred; description of constituents in chemical terms refers to the constituents at the time of addition to any combination specified in the description, and does not necessarily preclude chemical interactions among the constituents of a mixture once mixed; the first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

It is also to be understood that this invention is not limited to the specific embodiments and methods described below, as specific components and/or conditions may, of course, vary. Furthermore, the terminology used herein is used only for the purpose of describing particular embodiments of the present invention and is not intended to be limiting in any way.

It must also be noted that, as used in the specification and the appended claims, the singular form "a", "an", and "the" comprise plural referents unless the context clearly indicates otherwise. For example, reference to a component in the singular is intended to comprise a plurality of components.

The term "subject" refers to a human or animal, including all mammals such as primates (particularly higher primates), sheep, dog, rodents (e.g., mouse or rat), guinea pig, goat, pig, cat, rabbit, and cow. A subject is sometimes referred to herein as a "patient."

The term "cancer" refers to a disease or disorder characterized by uncontrolled division of cells and the ability of these cells to spread, either by direct growth into adjacent tissue through invasion, or by implantation into distant sites by metastasis. Exemplary cancers include, but are not limited to, primary cancer, metastatic cancer, carcinoma, lymphoma, leukemia, sarcoma, mesothelioma, glioma, germinoma, choriocarcinoma, prostate cancer, lung cancer, breast cancer, colorectal cancer, gastrointestinal cancer, bladder cancer, pancreatic cancer, endometrial cancer, ovarian cancer, melanoma, brain cancer, testicular cancer, kidney cancer, skin cancer, thyroid cancer, head and neck cancer, liver cancer, esophageal cancer, gastric cancer, intestinal cancer, colon cancer, rectal cancer, myeloma, neuroblastoma, pheochromocytoma, and retinoblastoma.

The term "therapeutically effective amount" means a dosage sufficient to reduce the level of IGF-1 in the subject. Such dosages may be administered by any convenient route, including, but not limited to, oral, parenteral, transdermal, sublingual, or intrarectal.

The term "GH/IGF-1 axis" as used herein refers to the endocrine system which regulates GH secretion and circulating IGF-1 levels. Growth hormone (GH) is secreted by somatotroph cells within the anterior pituitary gland. Neurosecretory nuclei of the hypothalamus Growth hormone stimulates the liver and other peripheral tissues to secrete insulin-like growth factor 1 (IGF-1). Peptides released by neurosecretory nuclei of the hypothalamus control the secretion of growth hormone. U.S. Pat. Appl. No. 20040121407 provides a description of the GH/IGF-1 axis.

The term "oxidative stress" refers to a biological state in which there is an overproduction of reactive oxygen species such that a biological system is unable to effectively detoxify reactive intermediates or repair resulting damage.

The term "oxidative damage" refers to the damage to biological tissue or compounds (i.e., DNA) caused by oxidative stress.

The term "population" refers to a group of subjects from which samples are taken for statistical measurement. For example, a population may be a group of subjects characterized by being within a predetermined age range, a group of all male subjects, a group of all female subjects, the group of all people in the United States, and the like.

In an embodiment of the present disclosure, a method of inhibiting development of a symptom aging (e.g., a symptom of an age related disease) in a subject is provided. The method comprises identifying a subject that does not suffer from acromegaly of less than 70 years of age with IGF-I levels in the highest quartile of a population and then administering a therapeutically effective amount of a GH/IGF-1 Axis inhibitory composition to the subject so that IGF-I levels are reduced to the median level for that population. Typically, the levels of IGF- 1 and insulin in the subject are monitored.

The present invention provides a GH/IGF-1 Axis inhibitory composition for use in a method for alleviating a symptom of chemotherapy in a subject having cancer as set out in the claims. Chemotherapy is known to cause various deleterious side effects some of which are caused by oxidative damage. Examples of side effects include weight loss, hair loss, gastrointestinal disturbances, alteration of blood chemistry and composition, immune suppression and the like. The method of this embodiment comprises identifying a subject undergoing chemotherapy and then administering a therapeutically effective amount of a GH/IGF-1 Axis inhibitory composition to the subject. Typically, the levels of IGF-1 and/or GH in the subject are monitored as well as chemotherapy related symptoms.

In another embodiment of the present disclosure, a method for reducing oxidative damage in a subject is provided. The method comprises identifying a subject predisposed to oxidative damage. In a refinement, a subject with an IGF-I level in the upper half of the normal age- and sex-specific levels of IGF-I compared to an average for the general population general population (excluding subjects diagnosed with acromegaly) is identified and then administered a therapeutically effective amount of a GH/IGF-1 Axis inhibitory composition so that the levels fall to below the median. Typically, the levels of IGF-1 and insulin in the subject are monitored as well as oxidative damage -related symptoms.

Oxidative damage is known to occur in a number of biological situations in which the present embodiment is useful. For example, such damage occurs in subjects undergoing chemotherapy, in subjects predisposed to or exhibiting symptoms of diabetes, in subjects predisposed to or exhibiting symptoms of stroke, and in subjects predisposed to cancer.

In another embodiment of the present disclosure, a method for inhibiting a symptom of aging and/or the onset of age related diseases (including Alzheimer's disease and stroke) is provided. The method comprises identifying a subject with an IGF-I level in the upper half of the normal age- and sex-specific levels of IGF-I compared to an average for the general population (for example,excluding subjects diagnosed with acromegaly) and then administering a therapeutically effective amount of a GH/IGF-1 Axis inhibitory composition to the subject so that the levels fall to below the median. Typically, the levels of IGF-1 and insulin in the subject are monitored.

In another embodiment of the present disclosure, a method for inhibiting the development of a symptom of cancer or the risk of cancer in a subject is provided. The method comprises identifying a subject with an IGF-I level in the upper half of the normal age- and sex- specific levels of IGF-I compared to an average for the general population (for example, excluding subjects diagnosed with acromegaly) and then administering a therapeutically effective amount of a GH/IGF-1 Axis inhibitory composition to the subject so that the levels fall to below the median. Typically, the levels of IGF-1 and insulin in the subject are monitored.

In another embodiment of the present disclosure, a method for inhibiting the development of a symptom of diabetes or the risk of diabetes in a subject is provided. The method comprises identifying a subject with an IGF-I level in the upper half of the normal age- and sex-specific levels of IGF-I compared to an average for the general population (for example, excluding subjects diagnosed with acromegaly) and then administering a therapeutically effective amount of a GH/IGF-1 Axis inhibitory composition to the subject so that the levels fall to below the median. Typically, the levels of IGF-1 and insulin in the subject are monitored.

In still another embodiment of the present disclosure, a method for reducing oxidative damage in various eukaryotic cells is provided. The method comprises identifying a eukaryotic cell predisposed to oxidative damage and then administering a therapeutically effective amount of a GH/IGF-1 Axis inhibitory composition to the subject.

In several of the embodiments set forth above, levels of IGF-1 and/or GH are measured to monitor and adjust the dosing for the subject. The levels of IGF-1 and GH are measured by any of a number of methods known in the art. Examples used to measure the level of IGH-1 in a subject include, but are not limited to, radioimmunoassay (RIA), ELISA (e.g., ELISA kits commercially available from Diagnostic Systems Laboratory, Inc., Webster, TX), chemiluminescent immunoassays (commercially available form Nichols Institute Diagnostic, San Juan Capistrano, CA).

As set forth above, the present invention utilizes a GH/IGF-1 Axis inhibitory composition as set out in the claims. Compositions that inhibit the GH/IGF-1 Axis are known and directly useful in the embodiments set forth above. In one variation, the GH/IGF-1 Axis inhibitory composition comprises a growth hormone receptor antagonist. Examples of growth hormone receptor antagonists are set forth in U.S. Pat. Nos. 5,849,535; 6,004,931; 6,057,292; 6,136,563; 7,470,779; 7,470,779; 7,524,813 and 6,583,115. The compositions set forth in these patents are generally growth hormone variants, which include several amino acid substitutions. In a refinement, the human growth hormone variant includes the following amino acid substitution: G120R. In another refinement, the human growth hormone variant includes at least one amino acid substitution selected from the group consisting of H18D, H21N, R167N, K168A, D171S, K172R, E174S, I179T, and G120R. In still another refinement, the human growth hormone variant includes the following amino acid substitutions: H18D, H21 N, R167N, K168A, D171S, K172R, E174S, I179T, and G120R. It should also be pointed out that these growth hormone variants are generally stabilized such as by being pegylated. A particularly useful specific example of a growth hormone receptor antagonist is Pegvisomant™ commercially available from Pfizer Inc. Pegvisomant™ is a recombinant 191 amino acid analog of the GH protein which has appended polyethyleneglycol groups (i.e., pegylation).

In another variation of the disclosure, the GH/IGF-1 Axis inhibitory composition comprises an IGF-I receptor antagonist.

In another variation of the disclosure, the GH/IGF-1 Axis inhibitory composition comprises a compound that inhibits the production of growth hormone. Such compounds typically act on the anterior pituitary gland. The commercially available compounds are synthetic variations of the naturally occurring somatostatin. Examples of these synthetic substitutes include octreotide (available as Sandostatin from Novartis Pharmaceuticals) and lanreotide (available as Somatuline from Ipsen).

In yet another variation of the disclosure, the GH/IGF-1 Axis inhibitory composition comprises a GH-releasing hormone (GHRH) receptor antagonist. An example of such an antagonist is MZ-5-156 (see, Effects of growth hormone-releasing hormone and its agonistic and antagonistic analogs in cancer and non-cancerous cell lines, N. Barabutis et al, International Journal of Oncology, 36: 1285-1289, 20100.

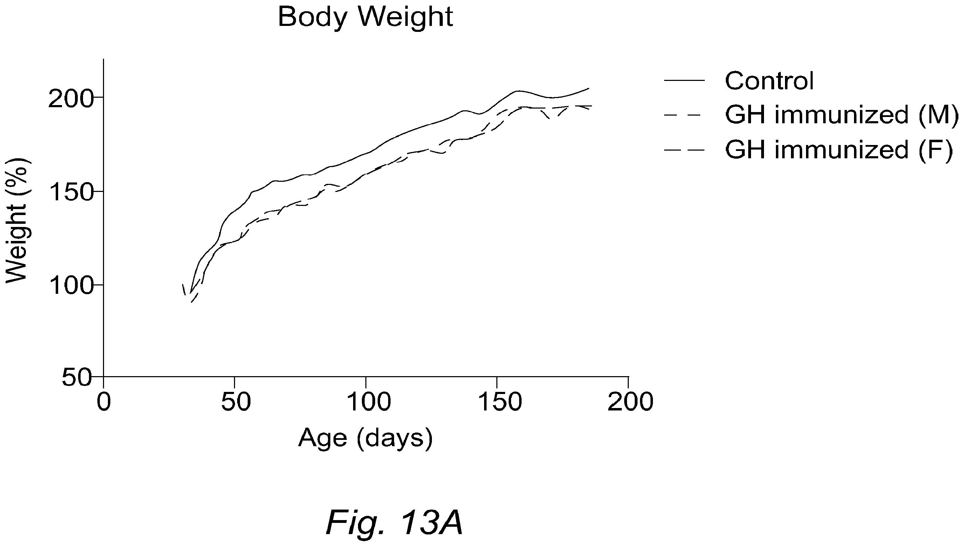

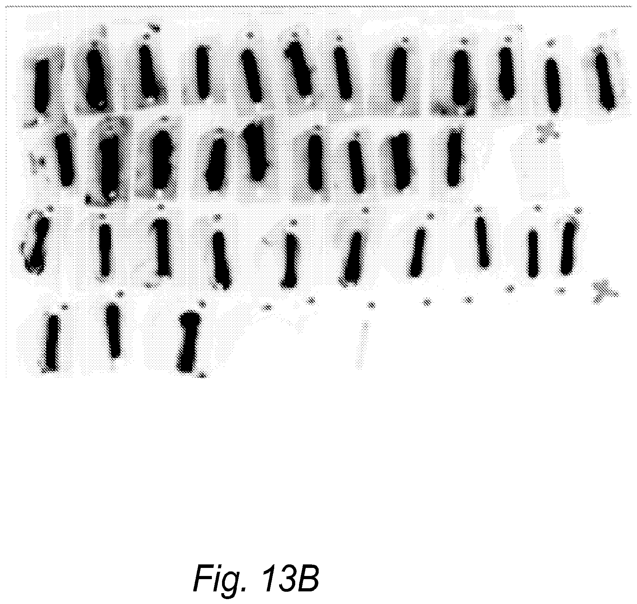

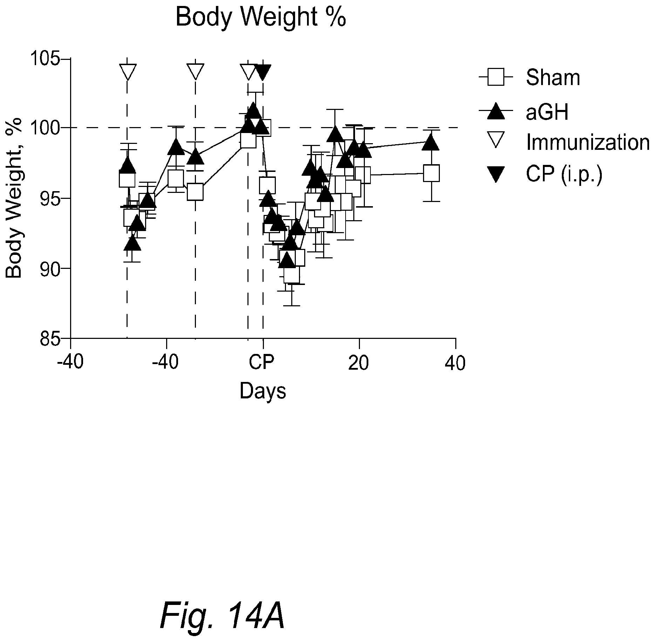

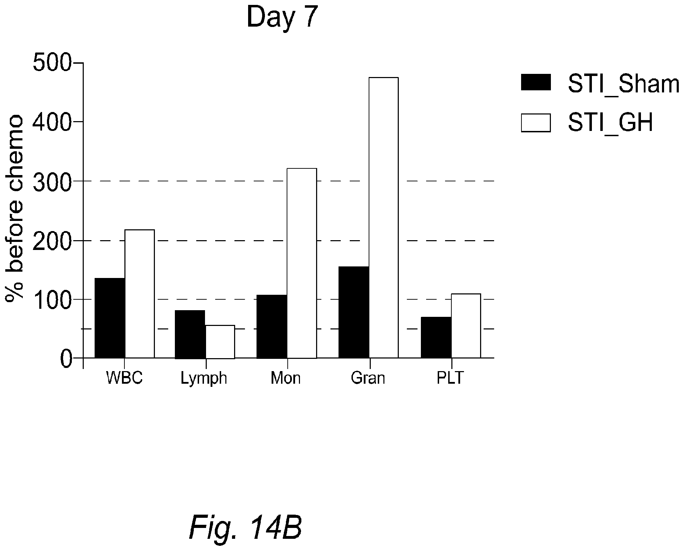

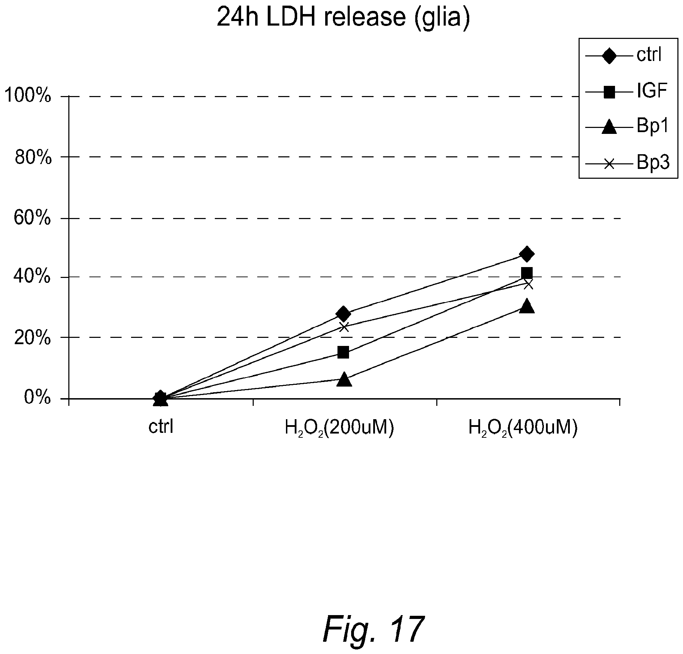

In another variation of the disclosure, the GH/IGF-1 Axis inhibitory composition comprises a growth hormone antibody. In one refinement, the growth hormone antibodies include monoclonal and polyclonal antibodies that target GH (See Fig. 13a, 13b, 14a, 14b), GHR (Fig. 17), or the IGF-1 receptor (IGF-IR). In a refinement, the monoclonal antibodies include immunoglobulins (e.g., IgGI and IgG2 subtypes). Examples of drugs incorporating these immunoglobulins include IMC-A12, R1507, AMG-479 (see reference below), SCH-717454, and CP-751,871 as set forth in the article Early drug development of inhibitors of the insulin-like growth factor-I receptor pathway: Lessons from the first clinical trials, by J. Rodon et al, Mol Cancer Ther 2008;7(9). September 2008, pages 2575-2588. In another refinement, the growth hormone antibodies include monoclonal and polyclonal antibodies that target growth hormone. AMG 479, a fully human anti -insulinlike growth factor receptor type I monoclonal antibody, inhibits the growth and survival of pancreatic carcinoma cells (Mol Cancer Ther May 2009 8: 1095-1105.)

In still another variation of the disclosure, the GH/IGF-1 Axis inhibitory composition comprises a combination of two or more of the possible selections set forth above.

The dose of the GH/IGF-1 Axis inhibitory composition is such that the measured level of plasma IGF-1 is lower than the subject's baseline level (value prior to treatment). Very low IGF-1 should be avoided as such low levels have related side effects. In one variation, the dose is adjusted such that the subject's plasma IGF-1 is from 20 to 60 percent of the subject's baseline level. In another variation, the dose is adjusted such that the subject's plasma IGF-1 is from 30 to 55 percent of the subject's baseline level. In still another variation, the dose is adjusted such that the subjects plasma IGF-1 is from 40 to 50 percent of the subject's baseline level. Normal values for IGF-1 concentration are dependent on age and on gender to some degree. A normal value for the IGF-1 level in a person in the 25 to 39 year range is from about 114 to 492 nanograms/ml (ng/ml), for a 40 to 54 year old person the normal range is from 90 to 360 ng/ml, and for a 55+ year old person the range is 71 - 290 ng/ml. For the elderly, the values are significantly lower while younger people may have higher values. In general, since many of the GH/IGF-1 Axis inhibitory compositions are currently used to treating several ailments, an initial dose in the context of the embodiments set forth above may be utilized. The dosing is then adjusted to achieve the target level of plasma IGF-1. In a refinement, the dose is adjusted by incremental adjusting in increments that are 20% to 60% of the initial dose. In another refinement, the dose is adjusted by incremental adjusting in increments that are 30% to 55% of the initial dose. In still another refinement, the dose is adjusted by incremental adjusting in increments that are 45% to 50% of the initial dose.

In the case of Pegvisomant™, the dosing recommended for treating acromegaly may be used as a starting dosing protocol. Therefore, a subcutaneous 40 mg loading dose is used followed by daily injections of 10 mg. The dose may be increased or decreased in 5 mg increments to achieve the IGF-1 levels set forth above.

In another embodiment, a kit encompassing one or more of the methods set forth above is provide. The kit includes a container having one or more doses of a GH/IGF-1 Axis inhibitory composition as set out in the claims. The kit also includes instructions indicating that the a GH/IGF-1 Axis inhibitory composition is to be provided to a subject (i.e., a subject at increased risk for cancer or a subject at increased risk for developing diabetes or a subject at first for oxidative damage) in accordance to methods and dosing regimens set forth above. In a refinement, the kit includes a vessel for holding a blood sample drawn from the subject to be used to monitor the levels of IGF-1 (and/or insulin in the case of diabetes).

The experiments set forth below confirm that the fundamental link between pro-growth pathway and age-dependent genomic instability observed in yeast, worms and mice studies is conserved in humans by reporting on a 22-year monitoring of an Ecuadorian cohort with growth hormone receptor and IGF-I deficiencies and investigating the effect of these deficiencies on the cellular response to stress and on markers of cancer and diabetes.

### RESULTS

### Ecuadorian cohort

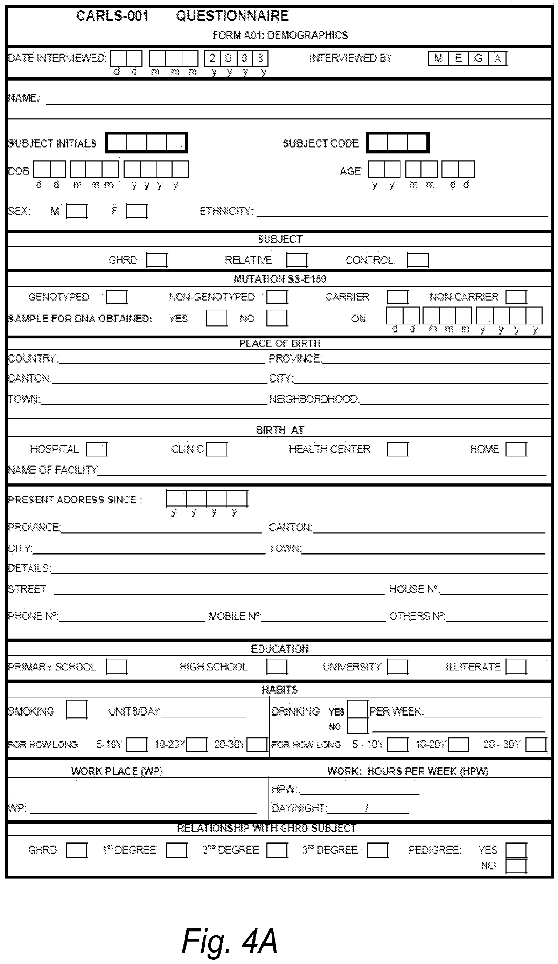

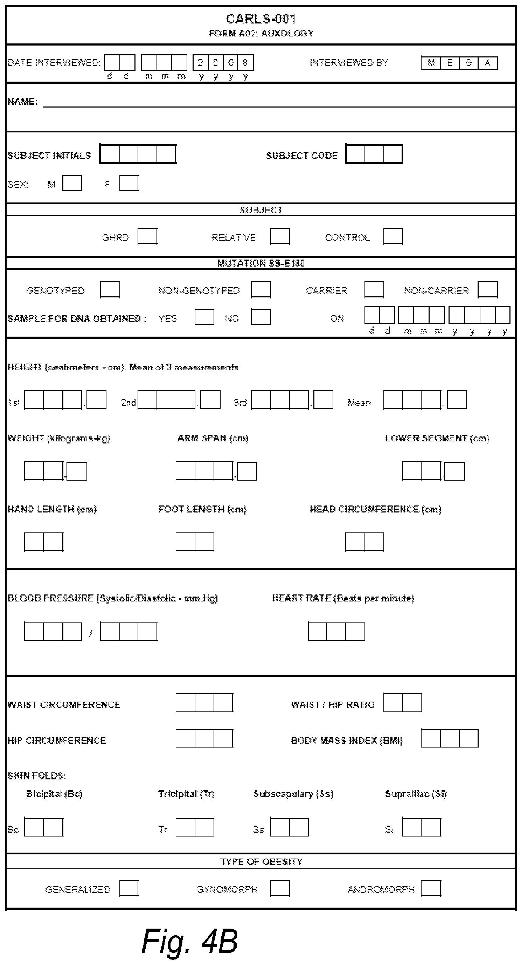

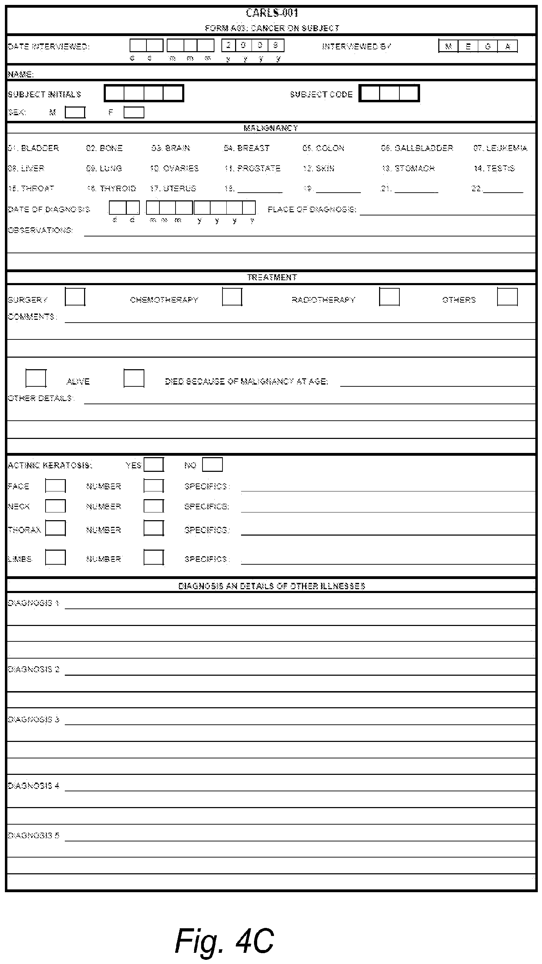

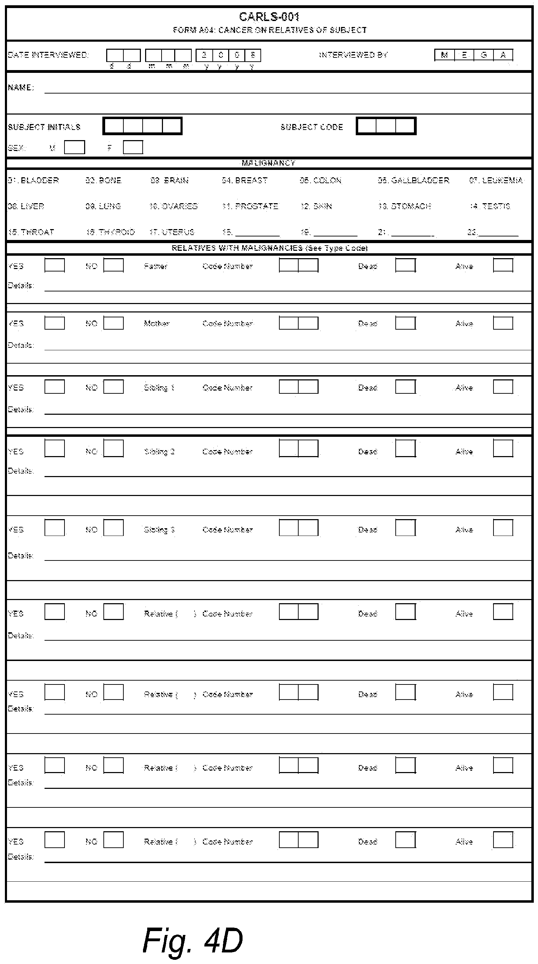

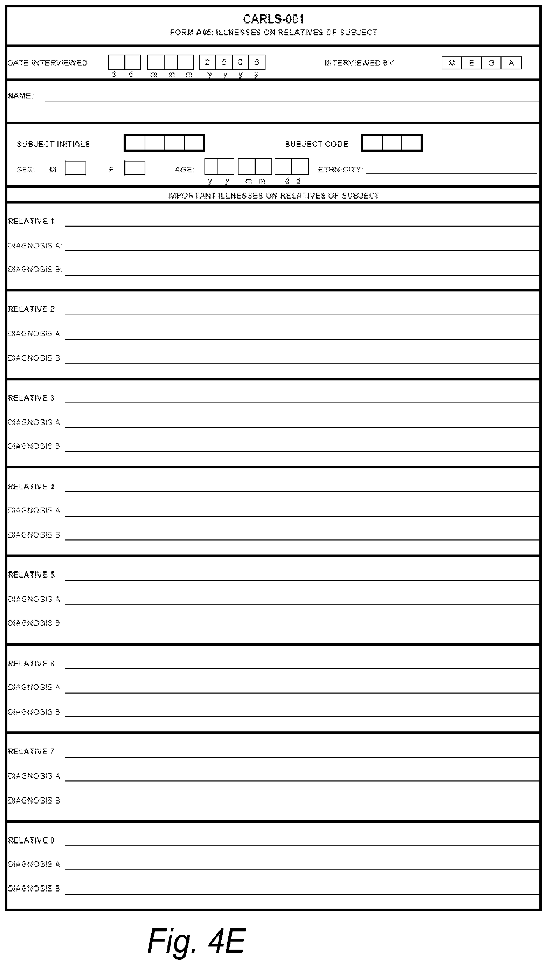

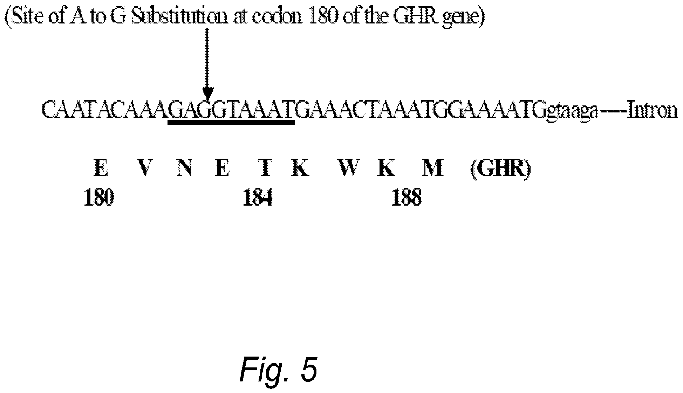

Study subjects were 99 individuals with Growth Hormone Receptor Deficiency (GHRD) who have been followed by one of the authors (J.G-A) at the Institute of Endocrinology, Metabolism and Reproduction (IEMYR) in Ecuador since 1988. Of these, 9 subjects died during the course of monitoring. The age distribution for 90 alive GHRD subjects and the control Ecuador population is shown in Fig. 1A (38). Using a questionnaire (Figures 4A-E), we collected mortality data for 53 additional GHRD subjects who died prior to 1988 and obtained information on illnesses and cause of death for 1606 unaffected first to fourth degree relatives (relatives) of the GHRD subjects. The GHRD cohort (99 subjects) was identified on the basis of their severe short stature of the subjects (*39-41*) and confirmed by genotyping (Fig. 1 B). The majority of GHRD subjects in this cohort were homozygous for an A to G splice site mutation at position 180 in exon 6 of the growth hormone receptor (GHR) gene (Fig. 1B, Fig. 5). This mutation, termed E180, results in a protein that lacks 8 amino acids in its extracellular domain and is possibly misfolded and degraded (Fig. 5) (*42*). Two GHRD subjects were homozygous for the R43X mutation, which results in a truncated GHR protein as a result of a premature stop codon (Fig. 1 B) (*43*) and two GHRD subjects were E180/R43X heterozygotes (Fig. 1 B). The E180 mutation is believed to have been introduced into this region by Spanish conversos who migrated to southern Ecuador in the early 1500s, and its predominance is attributed to the high level of consanguinity in this cohort (*44*, *45*). The R43X mutation occurs at a CpG dinucleotide hot spot and has been reported in subjects from around the world (*46*).

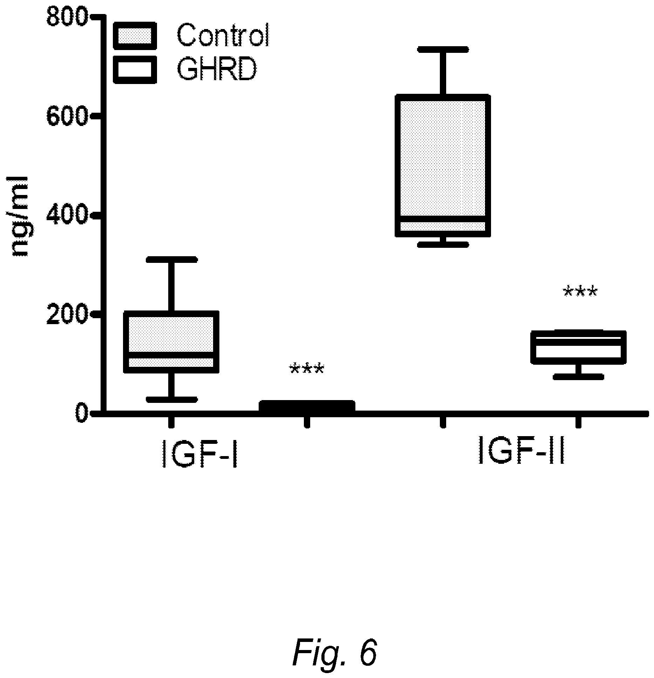

To confirm IGF deficiency in this cohort, we measured IGF-I and IGF-II concentrations (Fig. 6) in 13 control and 16 GHRD subjects ranging in age from 20-50 years, including those whose serum was later used for *in vitro* studies. Serum IGF-I ranged from 29 to 310 ng/ml (mean 144) among control subjects, but was ≤ 20 ng/ml in all GHRD subjects. Serum IGF-II ranged from 341-735 ng/ml (mean 473) among control subjects, but was below 164 ng/ml in all GHRD subjects (Fig. 6). There was no overlap in the range of IGF-I and IGF-II serum values between GHRD and control subjects (p<0.0001) (Fig. 6).

High mortality from common diseases of childhood has been observed in the GHRD cohort (Fig. 1 C) (*47*). Because of this, we only considered individuals who survived to at least age 10 for further analysis of diseases in this cohort. Of the 30 deaths among GHRD subjects (data from both monitoring and surveys) over the age of ten, 9 were due to age-related diseases (8 cardiac diseases, 1 stroke) and 21 were due to other causes (chagas disease, unknown). Compared to control individuals, GHRD subjects died much more frequently from accidents, alcohol-related causes and convulsive disorders (Fig. 1 D).

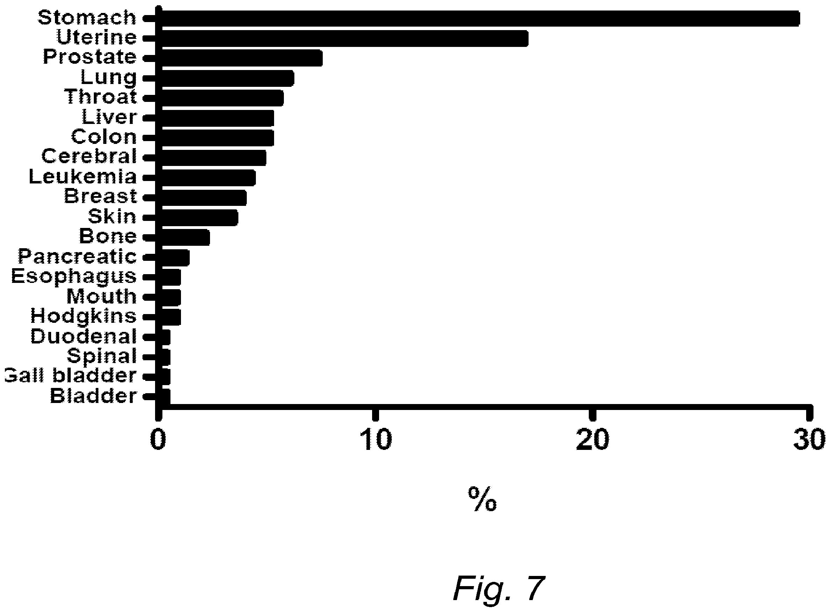

Cancer was not a cause of death in GHRD subjects of any age group (Fig.1 E); however, it accounted for approximately 20% of deaths and 17% of all diseases in the relatives (Fig.1D, F). Among deaths in each age-group, the proportion from cancer was lower in the GHRD subjects than in relatives (based on the exact hypergeometric distribution as implemented in StatXact 7, CytelSoftware Corporation, p=0.003). Of all the GHRD subjects monitored since 1988, only one was diagnosed with cancer, a papillary serous epithelial tumor of the ovary in 2008. After surgery and treatment, she remains cancer free. Stomach cancer was the predominant cause of cancer related mortality in the relatives (Fig. 7), which is consistent with the high incidence of this cancer in Ecuador (*48*).

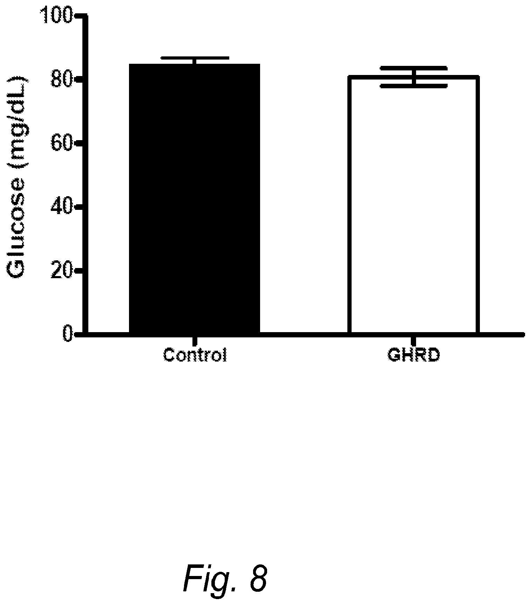

We did not observe any mortality or morbidity due to Type 2 diabetes in the GHRD cohort, whereas diabetes is responsible for 5% of deaths and 6% of all diseases in the relatives (Fig. 1D, F), in agreement with the 5% prevalence of diabetes in Ecuador (Fig. 1 G) (*49*). We estimated the prevalence of diabetes in the GHRD cohort as 0/90 = 0%, with 95% exact Clopper-Pearson Confidence Interval: 0% - 4%. To test whether the diabetes prevalence in the GHRD cohort was different from the general population prevalence of 5%, we performed an exact test of the null hypothesis that p=0.05, based on the Binomial distribution, with the type I error rate, α=0.05. The P-value was 0.02, indicating that the prevalence in the GHRD cohort is less than 5%. This is a particularly striking result considering the elevated prevalence of obesity among these GHRD individuals (21% in GHRD subjects *vs.* 13.4% in the general Ecuador population) (Fig.1 G). Hypoglycemia has been reported in children with GH deficiencies and young GHRD subjects (*50-52*). On the other hand, GH deficiency in adults is reported to cause insulin resistance and higher mortality from vascular disease (*36, 53*). To investigate the mechanisms that could be responsible for the observed lack of diabetes in the Ecuadorian GHRD cohort, we measured fasting glucose and insulin concentrations in 13 control and 16 GHRD subjects consisting of both male and female subjects between the ages of 20 and 50. We observed no significant difference in fasting glucose concentrations between them (Fig. 8). However, the average insulin concentration in the GHRD group was approximately a third of that in controls (Fig.1H, p<0.05), and the homeostasis model assessment of insulin resistance (HOMA-IR) index (*54*) indicated that GHRD subjects (HOMA-IR =0.34) were much more insulin sensitive than control subjects (HOMA-IR=0.96) (Fig. 1I, p<0.05) (*55*)*.* These results are consistent with the finding that GHRD mice and other GH deficient mouse models have low serum insulin concentrations and are insulin sensitive (*31-34*).

Although GHRD subjects may have elevated cardiac disease mortality compared to unaffected relatives (Fig. 1 D), relative mortality from vascular diseases (combining cardiac disease and stroke) appears to be similar to relatives (33% of deaths in relatives *vs.* 30% of deaths in GHRD subjects) because only 3% of the deaths in GHRD subjects *vs* 12% in the relatives were caused by strokes (Fig. 1 D). In agreement with studies of a human population with isolated growth hormone deficiency (IGHD) (*56*), our data suggest that GHRD does not increase overall vascular disease mortality, although it may increase susceptibility to cardiac disease while decreasing susceptibility to stroke (Fig. 1 D).

### Reduced IGF-1 signaling protects against DNA damage and favors apoptosis of damaged cells.

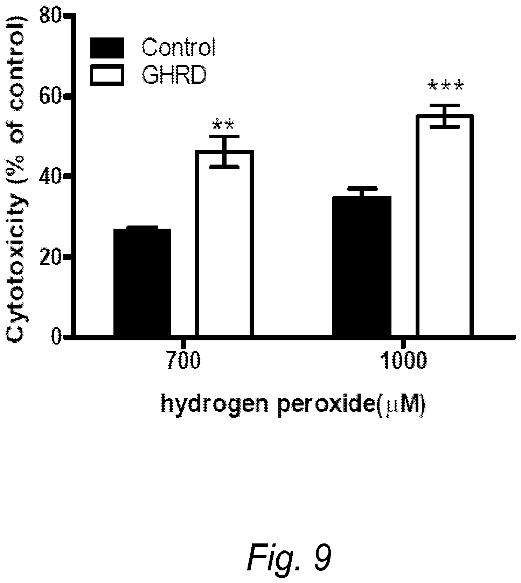

The role of IGF-I in tumor development and progression has been attributed to promotion of cell growth and inhibition of apoptosis in damaged and pre-cancerous cells (*29*). However, our studies in *S. cerevisiae* indicate that homologs of mammlian growth signaling pathway genes, including *TOR*, *S6K*, *RAS* and *PKA* promote an age-dependent increase in DNA mutations by elevating superoxide production and promoting DNA damage independently of cell growth (*20*). In fact, the mutation spectrum in p53 from human cancers is similar to that in aging yeast (*19*, *20, 28*). This raises the possibility that GH and IGF-I signaling may promote mutations and cancer not only by preventing apoptosis of damaged cells but also by increasing DNA damage in both dividing and non-dividing cells. To test this hypothesis, we incubated confluent HMECs in medium supplemented with 15% serum from either controls or GHRD subjects (*57, 58*) for 6 hours and then treated them with H₂O₂ for 1 or 24 hours, followed by comet analysis to detect DNA strand breaks. In order to prevent interference from growth factors or insulin the medium did not contain any growth supplements during the 6-hour incubation period. Because cells were incubated to greater than 90% confluence, cell growth during the pre-incubation and H₂O₂ treatment periods was minimal. Six serum samples were independently tested for each group. Comet analysis indicated that cells incubated in serum from GHRD subjects had fewer DNA breaks after treatment with 700µM H₂O₂ for 1 hour (Fig. 2A, B) or 24 hours (Fig. 2A, C), suggesting that serum from GHRD subjects can protect against oxidative DNA damage independently of cell division. We also incubated confluent epithelial cells in medium supplemented either with control serum, GHRD serum, or GHRD serum supplemented with 200ng/ml IGF-I for 6 hours (normal levels of IGF-I in Ecuadorian human adults range between 96-270 ng/ml) (*39*). Although 100µM H₂O₂ had a similar cytotoxic effect in cells incubated in GHRD serum and those incubated in control serum or GHRD serum + 200 ng/ml IGF-I (Fig. 2 D), treatment with 700µM H₂O₂ resulted in higher cytotoxicity in cells incubated in GHRD serum than in control serum (Fig. 2 D). This effect was completely reversed by the addition of 200ng/ml IGF-I to GHRD serum (Fig. 2 D). Mouse embryonic fibroblast (MEF) cells were also more susceptible to increased cytotoxicity in response to H₂O₂ when incubated in GHRD serum rather than control serum (Fig. 9). Furthermore, HMECs incubated in GHRD serum and treated with H₂O₂ showed higher caspase activity than cells incubated in control serum, indicating the activation of apoptosis (Fig. 2 E), in agreement with the proposed role of IGF-I signaling in increasing cancer incidence by preventing apoptosis (*29*).

To test whether IGF-I receptor signaling was responsible for the sensitization of cells to oxidative damage, we analyzed DNA damage MEF cells lacking the IGF-I receptor (R- cells) or overexpressing the human IGF-I receptor (R+ cells) (*60*). R+ cells had higher basal DNA than did R- cells (Fig. 2 F). Western blot analysis confirmed the anticipated increase in phosphorylation of Akt (Thr 308) and FoxO1 (Ser 256) in R+ cells compared to R- cells, indicating that Akt was activated while FoxO1 was inactivated in the R+ cells (Fig. 2 G) (*61*-*63*). The very low level of total FoxO1 protein in R+ cells may be due to the Akt-mediated phosphorylation of FoxO, which results in its ubiquitination and proteasomal degradation (Fig. 2 G) (*64*). Reduced FoxO activity in R+ cells when compared to R- cells that were transfected with a FoxO luciferase reporter plasmid confirmed that FoxO was inactivated by high IGF-I signaling in these cells (Fig. 2 H). As FoxO transcription factors are known to protect against oxidative stress as well as promote apoptosis (*62, 65, 66*), we hypothesize that increased FoxO activity could account, in part, for the protective effects observed in R- cells and in HMECs incubated in GHRD serum. In fact, microarray analysis of epithelial cells incubated in either control or GHRD serum showed that out of 44 genes that were significantly upregulated in the GHRD serum group, 4 genes, including *SOD2*, were FoxO targets (Fig. 2 I).

### Protective effects of reduced pro-growth signaling in yeast and mammals

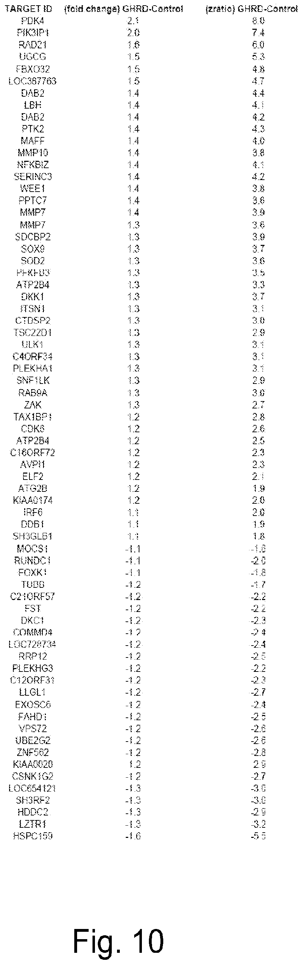

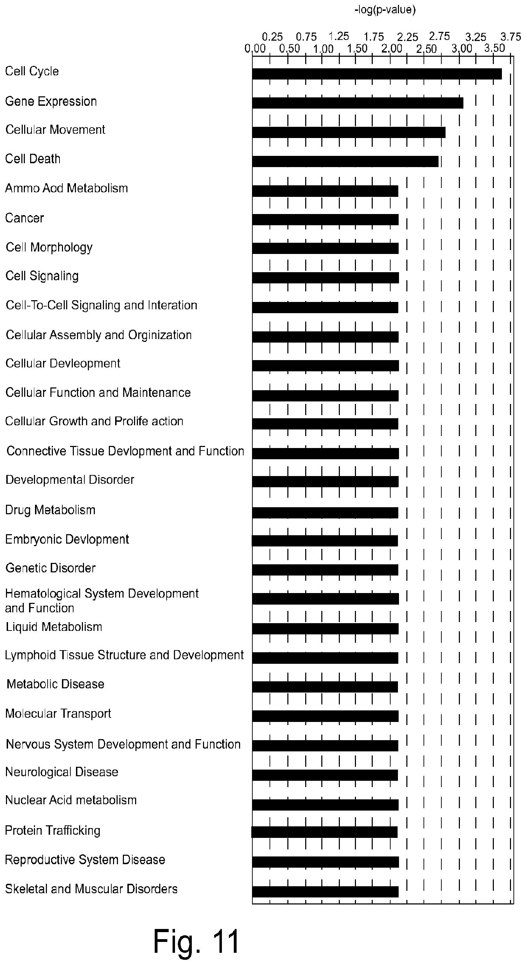

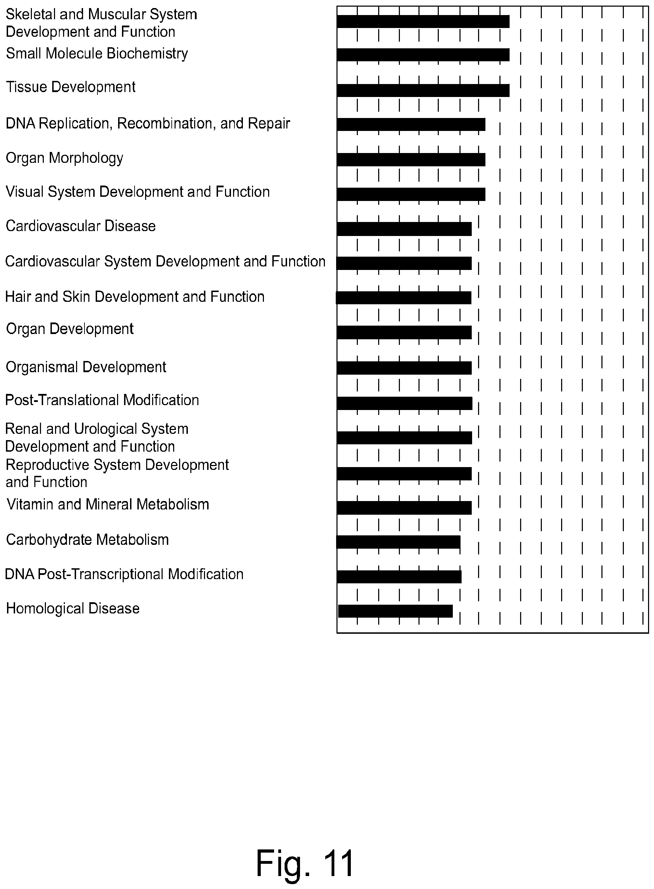

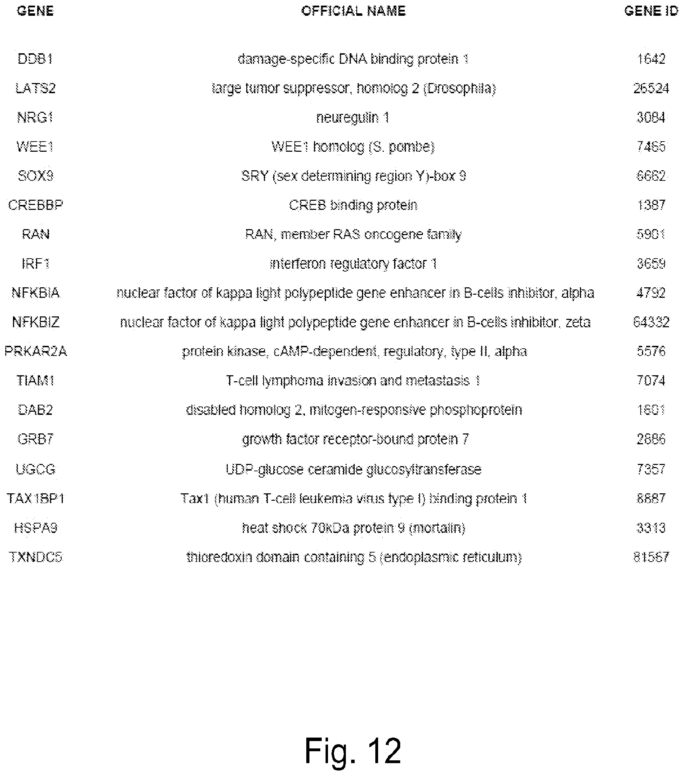

A complete list of genes with significant differential expression in HMECs incubated in either control or GHRD serum is shown in the table of Figure 10. Ingenuity Pathways Analysis (IPA) (*67*) of global gene expression patterns revealed significant differences in pathways involved in cell cycle regulation, gene expression, cell movement and cell death, among others (Fig. 11-12). IPA also indicated that genes regulating apoptosis were upregulated and Ras, PKA and Tor signaling were downregulated in cells incubated in GHRD serum. RT-PCR analysis confirmed a 1.3 times higher steady state mRNA level of mitochondrial MnSOD (SOD2) in cells incubated in GHRD serum, and also a 70%, 50% and 20% reduction in N-Ras, PKA and TOR expression, respectively (Fig. 3 A). Ras, PKA and TOR/S6K are central regulators of pro-aging and disease promoting pathways (68) and SOD2 is a key mediator of cellular protection against oxidative stress in organisms ranging from the unicellular yeast to mammals (*2, 19*, *20, 69-71*).

To further test the role of these genes in age and oxidative stress-dependent DNA damage, we generated a yeast triple mutant strain lacking Ras, Tor1 and Sch9. Our previous studies have shown that yeast *sch9*Δ mutants exhibit lower age-dependent genomic alterations than wild-type cells in part due to reduced error-prone Polζ-dependent DNA repair (*20*). We observed a major life span extension in non-dividing triple mutant cells compared to wild type cells (Fig. 3 B). We analyzed age-dependent DNA genomic instability in the *ras2A tor1 Δ sch9 Δ* and wild type cells by measuring the frequency of mutations of the *CAN1* gene. The *CAN1* gene encodes a high affinity arginine permease involved in the uptake of arginine but also of its analog canavanine, which is toxic to the cells (*19*). Mutations that inactivate *CAN1* gain the ability to form a colony on minimal medium containing canavanine. The frequency of age-dependent mutations in the *CAN1* gene, which are mostly point mutations including a high frequency of G to T (transversion) and C to T (transition) base substitutions (*19*), were much higher in wild type cells compared to the triple mutants (Fig. 3 C). Whereas wild-type cells were susceptible to H₂O₂ treatment, the *ras2*Δ *tor1*Δ *sch9*Δ mutants were almost unaffected at the concentrations tested (Fig. 3 D). This was accompanied by a significant increase in age- and oxidative stress-dependent mutations in wild-type but not in the long-lived *ras2Δ tor1Δ sch9Δ* mutants (Fig. 3 E). These results show that yeast cells lacking homologs of genes downregulated in HMECsexposed to GHRD serum (Fig. 3 F), exhibit a remarkable decrease in DNA mutations and a major life span extension.

### DISCUSSION

The reduced incidence of age-related pathologies in GHRD subjects is consistent with studies in mice showing that close to 50% of GHRD or GH deficient animals die without any obvious evidence of age-related pathological lesions, compared to only about 10% of their wild-type siblings although GHRD mice can live 40% longer (*23*)(*14, 22, 23, 77*). In agreement with the results presented here, GHRD mice display a lower incidence (49%) and delayed occurrence of fatal neoplasms compared with their wild-type littermates, increased insulin sensitivity, and a reduction in age-dependent cognitive impairment (*23, 24, 31*). Similar phenotypes are also observed in GH deficient mice (*22, 32*). Furthermore, the reduced cancer incidence in GHRD mice is associated with a lower mutation frequency in various tissues (*25*).

Unlike in mouse models, GHRD does not appear to extend the human lifespan, in large part because 70% of the deaths in this cohort are caused by non age-related causes including convulsive disorders, alcohol toxicity, accidents, liver cirrosis and other unknown causes *vs* the generally normal distribution of causes of death in the cohort of relatives. The lack of cancer mortality but normal life span in subjects with reduced growth hormone signaling in this study are in agreement with a preliminary study by Shevah and Laron that reported the absence of cancer in a group of 222 patients with congenital IGF-I deficiencies (*73*) and those of Aguiar-Oliveira et al., who reported normal longevity in 65 GH deficient subjects (*74*). In contrast to our study which focuses on GHRD subjects with specific mutations and compares them to age-matched relatives, in their study, Shevah and Laron compared young subjects in which IGF-I deficiency was caused by many causes with much older controls which made it difficult to interpret the data. However, together, these two studies provide strong evidence to suggest reduced cancer incidence in GHR and IGF-I deficient subjects and indicate that IGF-I could serve as a marker for age-dependent cancer, at least in specific populations. Our results may also provide a partial explanation for the overrepresentation of partial loss-of-function mutations in the IGF-1 receptor gene among Ashkenazi Jewish centenarians (*75*).

The mechanisms of IGF-I pro-cancer role may involve its well established role in promoting growth and inhibiting apoptosis (*29, 76, 77*) but also its counterintuitive effect on increasing DNA damage independently of growth as suggested by our studies in yeast. In both yeast and mammals, reduction of TOR/S6K, RAS and AC/PKA signaling renders cells and the organism resistant to aging and oxidative stress-dependent mutagenesis (*2, 19, 20, 78-80*). This effect appears to depend, in part, on increased activity of stress resistance transcription factors and SOD2 (*20, 65, 81*). In fact, mice lacking Cu/Zn SOD or MnSOD are susceptible to increased DNA damage and cancer (*71*). The effect of serum from GHRD subjects in promoting many of the changes that promote longevity in model organisms, including reduced levels of RAS, PKA, and TOR and increased expression of FOXO-regulated genes including SOD2, raises the possibility that the anti-aging and anti-DNA damage mechanisms promoted by reduced growth signaling are conserved from yeast to humans.

The lack of type 2 diabetes in the GHRD cohort is particularly interesting considering that the clinical phenotype of subjects with GHRD includes obesity (*82*). The enhanced insulin sensitivity of GHRD subjects, as indicated by reduced insulin concentrations and a lower HOMA-IR index, could explain the absence of diabetes in this cohort. Although increased insulin sensitivity has been associated with a longer lifespan in mouse models (*83*), some long-lived mice, including the fat insulin receptor knockout (FIRKO) mice, exhibit impaired insulin signaling. In this case however, loss of insulin signaling is restricted to adipose tissue and is not associated with diabetes or glucose intolerance (*84*). Similarly, male IGF-I receptor heterozygous mice show a 15% increase in lifespan although they exhibit impaired glucose tolerance (*6*).

### MATERIALS AND METHODS

**Subject Recruitment:** GHRD subjects and relatives were recruited for the study under protocols approved by the Institute for Endocrinology, Metabolism and Reproduction (IEMYR) in Ecuador. All participants signed informed consent forms prior to their participation in the study. Data on deceased GHRD subjects was collected by interviewing family members using a detailed questionnaire (Fig.A-E). At least two relatives were required to be present at the time of the interview.

**Genotyping:** Saliva samples were collected using the Oragene OG-250 DNA collection kit (DNA Genotek Inc., Ontario, Canada) and processed according to the manufacturers protocol. Genotyping of the E180 mutation was done using the following primers -
SEQ ID NO 3: 5'-CATTGCCCTCAACTGGACTT-3' Forward
SEQ ID NO 4: 5'-CATTTTCCATTTAGTTTCATTTACT-3' Reverse (WT)
SEQ ID NO 5: 5'-CATTTTCCATTTAGTTTCATTTAC-3' Reverse (mutant)

**Serum Analysis:** Serum IGF-I and IGF-II were measured using an in-house ELISA based assay developed at UCLA. Briefly, serum samples were extracted with acid/ethanol and added to 96 well microtiter plates (50ul/well) that had been precoated with IGF-I or IGF-II monoclonal antibodies (R& D systems) at a concentration of 0.5µg/well. Following a 2 hour incubation and subsequent wash, 100µl of streptavidin-HRP conjugate was added to each well and incubated for 20min. 100µl of OPD substrate was added to each well and further incubated for 10-20 min. The reaction was stopped by the addition of 2N H₂SO₄ and absorbance was measured at 490nm with a plate reader (Molecular Design). Values were calculated against known IGF-I and IGF-II standards. Fasting glucose levels were measured with a glucose analyzer from YSI Life Sciences and fasting insulin levels were measured with a human insulin ELISA kit from Millipore. Insulin resistance was assessed using the homeostatic model assessment-insulin resistance (HOMA-IR) index from fasting glucose and fasting insulin values, and calculated with the formula, fasting glucose (mg/dL) x fasting insulin (µU/ml) /405 (*54*).

**Cell culture:** HMECs were purchased from ScienCell Research Laboratories. Cells were cultured in epithelial cell medium (ScienCell) at 37°C and 5% CO₂ on poly-L-lysine (Sigma) coated culture dishes. The epithelial cell medium consisted of basal medium and a proprietary growth supplement supplied by the manufacturer. Primary mouse embryonic fibroblasts (MEFs) were purchased from ATCC (Manassas, VA) and cultured in DMEM/ F12 (Invitrogen), supplemented with 15% FBS at 37°C and 5% CO₂. R+ and R- cells were obtained from Dr. R. Baserga and cultured in DMEM/F12 supplemented with 10% FBS at 37°C and 5% CO₂. Cells were seeded at a density of 4x10⁴ per well for comet and apoptosis assays, 8x10⁴ per well for LDH assays or 2x10⁵ per well for microarray analysis and western blots in 24, 96 and 6 well plates respectively. Cells were grown in epithelial cell basal medium supplemented with 15% control or GHRD serum for 6 hours followed by treatment with H₂O₂ for 1 hour (comet and apoptosis assays) or 24 hours (comet and LDH assays). For microarray analysis, cells were grown in epithelial cell basal medium (Sciencell) and supplemented with control or GHRD serum for 6 hours, and immediately processed for RNA extraction with TRI reagent from Ambion.

**Comet Assay:** Comet assay was performed according to the method described by Olive et al (85) using the comet assay kit from Trevigen. DNA damage was quantified per cell with the Comet Score™ software. 100-200 cells were analyzed per sample.

**LDH assay:** LDH activity was assayed in culture medium with the CytoTox 96 Non-Radioactive Cytotoxicity Assay from Promega according to the manufacturer's protocol.

**Apoptosis assay:** Activated caspases were quantified with a fluorescence plate reader with the Fluorescein CaspaTag Pan-Caspase Assay Kit (Chemicon) according to the manufacturer's protocol.

**FoxO activity:** 50,000 cells/well were transfected with 0.2µg of FoxO luciferase reporter plasmid with the consensus FoxO binding sequence driving firefly luciferase gene expression in 24 well plates. As an internal control cells were co-transfected with 0.02µg of plasmid DNA encoding Renilla luciferase under control of the CMV promoter. 24 hours after transfection, FoxO promoter activity was assayed using the Dual-Luciferase Reporter Assay System from Promega according to the manufacturer's protocol.

**Western blot analysis:** Cells were lysed in RIPA buffer and total protein was assayed with BCA from Thermo scientific. 15 µg of total protein was loaded on denaturing 10% SDS-PAGE gels. Primary antibodies against phospho and total Akt (Thr 308) as well as phospho and total FoxO1 (Ser 256) were obtained from Cell Signaling Technologies. β-tubulin was obtained from Santa Cruz Biotechnology Inc. Secondary rabbit antibody was obtained from Jackson Immunoresearch Laboratories, Inc.

**Microarray analysis:** RNA was extracted using TRI Reagent (Ambion) according to protocol and hybridized to BD-103-0603 chips from Illumina Beadchips (San Diego, CA). Raw data were subjected to Z normalization as described (*86*) and are available at the gene expression omnibus (GEO) repository, accession number GSE21980. Gene set enrichment was tested with the PAGE method as described (*67*).

Figures 11, 13 and 14 were selected based on the names and descriptions provided by Ingenuity Pathways Analysis (Ingenuity Systems; Redwood City, CA) and/or Ariadne Pathway Studio 7 (Ariadne Genomics).

**Yeast:** Wild type DBY746 (*MATα,leu2-3,112,his3Δ1,trp1-289,ura3-52,GAL*⁺) and its derivative *ras2::LEU2tor1::HIS3sch9::URA3*, originated by one-step gene replacement according to Brachmann et al. (*87*), were grown in were grown in SDC containing 2% glucose and supplemented with amino acids as described (*88*), as well as a 4-fold excess of the supplements tryptophan, leucine, uracil, and histidine. Chronological life span in SDC medium was monitored by measuring colony forming-units (CFUs), on YPD plates, every other day. The number of CFUs on day one was considered to be the initial survival (100%) and was used to determine the age-dependent mortality (*89*). Spontaneous mutation frequency was evaluated by measuring the frequency of mutations of the *CAN1* (*YEL063C*) gene. Cells were plated onto selective SDC-Arginine plates in the presence of L-canavanine sulfate [60mg/L]. Mutation frequency was expressed as the ratio of Can^{r} colonies over total viable cells (*90*). Resistance to oxidative stress was also evaluated in yeast cultures chronically treated with 1 mM H₂O₂ on days 1 and 3. Percent of survival and *can1* mutation frequency were measured as described above.

**Statistical analysis:** Students two tailed t-test was used to analyze insulin, HOMA-IR data, and cellular data from mammalian (comet, LDH, caspase assays, RT-PCR, and FoxO activity) and yeast experiments (survival and mutation frequency) using graph pad prismV.

### Chemotherapy Experiments

The results of an experiment in which human Growth Hormone was administers to mice is set forth in Figure 13A. This figure provides a plot of body weight of mice immunized with human Growth Hormone (huGH) (20 male and 20 female) versus time. Lower body weight in these mice indicates that inhibitory antibodies against human GH have been generated by the mice. FIGURE 13B provides an image from a slot blot that shows the anti-huGH activity in the serum from immunized mice were used to blot the immobilized human GH. It is observed that 34 out of 40 mice immunized with huGH showed strong activity.

FIGURE 14A provides plots of the body weight of mice with short term immunization (STI) with human GH (half-filled arrowheads) after cyclophosphamide treatment (cyclophosphamide (CP) i.p., 300 mg/kg, indicated by solid arrowheads). FIGURE 14B provides a histogram of the complete blood count (CBC) 7-days after CP treatment (Data are presented as percentage of pre-chemo value). Figures 14A and 14B collectively show that the immunization (and therefore the antibodies produced) provide a protective effect against chemotherapy side effects.

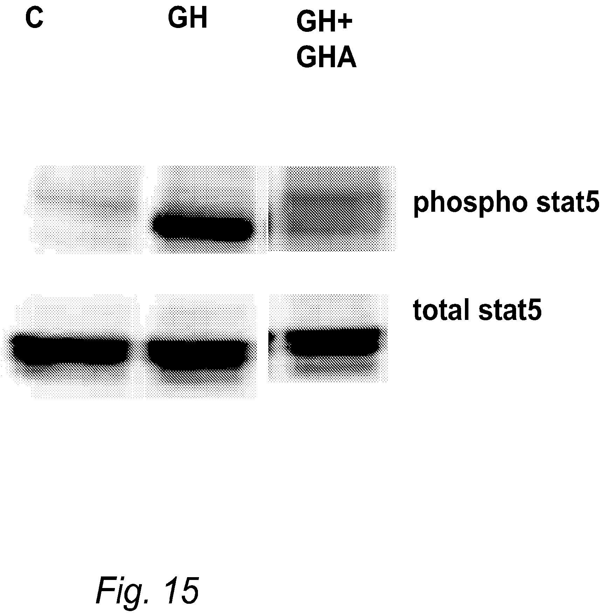

FIGURE 15 shows the efficacy of the Growth hormone receptor antagonist in blocking the growth hormone receptor activity. Mouse L cell fibroblasts expressing GHR were serum starved for 24 hours, then treated with 5nM growth hormone (GH) for 5 min either without or without a 30 min, 50nM growth hormone antagonist (GHA) pre-treatment. The phospho stat5 signal reflects the activity of the growth hormone receptor.

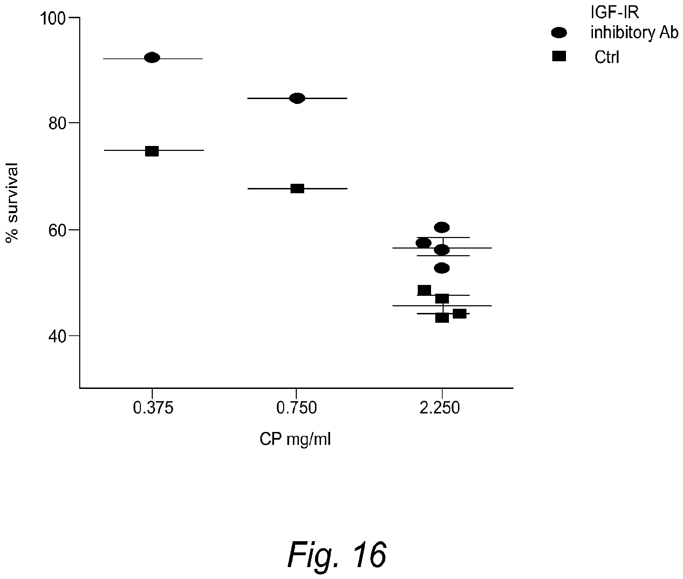

FIGURE 16 provides a plot of an experiment in which human stem cells from amniotic fluid were pre-treated with an inhibitory antibody that blocks the IGF-I receptor before treatment with different doses of the chemotherapy drug cyclophosphamide at the indicated doses. This figure shows that an antibody that blocks the growth hormone receptor protects cells against oxidative damage and chemotherapy.

FIGURE 17 provides a plot of the 24 hour lactate dehydrogenase (LDH) release from the treatment of primary glial cells with the IGF-I inhibitor insulin-like growth factor-binding protein 1 (IGFBP1) and with insulin-like growth factor-binding protein 2 (IGFBP2). It is observed that IGFBP1 protects them against oxidative damage. IGFBP1 is a strong IGF-I inhibtor. Other IGFBPs can even increase IGF-I signaling.

### REFERENCES AND NOTES

1. C. Kenyon, J. Chang, E. Gensch, A. Rudner, R. Tabtiang, A C. elegans mutant that lives twice as long as wild type. Nature 366, 461-464 (Dec 2, 1993).
2. P. Fabrizio, F. Pozza, S. D. Pletcher, C. M. Gendron, V. D. Longo, Regulation of longevity and stress resistance by Sch9 in yeast. Science 292, 288-290 (Apr 13, 2001).
3. M. Tatar et al., A mutant Drosophila insulin receptor homolog that extends life-span and impairs neuroendocrine function. Science 292, 107-110 (Apr 6, 2001).
4. D. J. Clancy et al., Extension of life-span by loss of CHICO, a Drosophila insulin receptor substrate protein. Science 292, 104-106 (Apr 6, 2001).
5. K. T. Coschigano et al., Deletion, but not antagonism, of the mouse growth hormone receptor results in severely decreased body weights, insulin, and insulin-like growth factor I levels and increased life span. Endocrinology 144, 3799-3810 (Sep, 2003).
6. M. Holzenberger et al., IGF-1 receptor regulates lifespan and resistance to oxidative stress in mice. Nature 421, 182-187 (Jan 9, 2003).
7. V. D. Longo, C. E. Finch, Evolutionary medicine: from dwarf model systems to healthy centenarians? Science 299, 1342-1346 (Feb 28, 2003).
8. V. D. Longo, E. B. Gralla, J. S. Valentine, Superoxide dismutase activity is essential for stationary phase survival in Saccharomyces cerevisiae. Mitochondrial production of toxic oxygen species in vivo. J Biol Chem 271, 12275-12280 (May 24, 1996).
9. V. D. Longo, Thesis. University of California, Los Angeles, (1997).
10. J. Z. Morris, H. A. Tissenbaum, G. Ruvkun, A phosphatidylinositol-3-OH kinase family member regulating longevity and diapause in Caenorhabditis elegans. Nature 382, 536-539 (Aug 8, 1996).
11. K. Lin, J. B. Dorman, A. Rodan, C. Kenyon, daf-16: An HNF-3/forkhead family member that can function to double the life-span of Caenorhabditis elegans. Science 278, 1319-1322 (Nov 14, 1997).
12. W. C. Orr, R. S. Sohal, Extension of life-span by overexpression of superoxide dismutase and catalase in Drosophila melanogaster. Science 263, 1128-1130 (Feb 25, 1994).
13. K. Flurkey, J. Papaconstantinou, D. E. Harrison, The Snell dwarf mutation Pit1(dw) can increase life span in mice. Mech Ageing Dev 123, 121-130 (Jan, 2002).
14. H. M. Brown-Borg, K. E. Borg, C. J. Meliska, A. Bartke, Dwarf mice and the ageing process. Nature 384, 33 (Nov 7, 1996).
15. E. Cohen et al., Reduced IGF-1 signaling delays age-associated proteotoxicity in mice. Cell 139, 1157-1169 (Dec 11, 2009).
16. V. D. Longo, L. L. Liou, J. S. Valentine, E. B. Gralla, Mitochondrial superoxide decreases yeast survival in stationary phase. Arch Biochem Biophys 365, 131-142 (May 1, 1999).
17. C. Kenyon, A conserved regulatory system for aging. Cell 105, 165-168 (Apr 20, 2001).
18. V. D. Longo, Mutations in signal transduction proteins increase stress resistance and longevity in yeast, nematodes, fruit flies, and mammalian neuronal cells. Neurobiol Aging 20, 479-486 (Sep-Oct, 1999).
19. F. Madia et al., Longevity mutation in SCH9 prevents recombination errors and premature genomic instability in a Werner/Bloom model system. J Cell Biol 180, 67-81 (Jan 14, 2008).
20. F. Madia et al., Oncogene homologue Sch9 promotes age-dependent mutations by a superoxide and Rev1/Polzeta-dependent mechanism. J Cell Biol 186, 509-523 (Aug 24, 2009).
21. J. M. Pinkston, D. Garigan, M. Hansen, C. Kenyon, Mutations that increase the life span of C. elegans inhibit tumor growth. Science 313, 971-975 (Aug 18, 2006).
22. Y. Ikeno, R. T. Bronson, G. B. Hubbard, S. Lee, A. Bartke, Delayed occurrence of fatal neoplastic diseases in ames dwarf mice: correlation to extended longevity. J Gerontol A Biol Sci Med Sci 58, 291-296 (Apr, 2003).
23. Y. Ikeno et al., Reduced incidence and delayed occurrence of fatal neoplastic diseases in growth hormone receptor/binding protein knockout mice. J Gerontol A Biol Sci Med Sci 64, 522-529 (May, 2009).
24. A. Bartke, Insulin resistance and cognitive aging in long-lived and short-lived mice. J Gerontol A Biol Sci Med Sci 60, 133-134 (Jan, 2005).
25. A. M. Garcia et al., Effect of Ames dwarfism and caloric restriction on spontaneous DNA mutation frequency in different mouse tissues. Mech Ageing Dev 129, 528-533 (Sep, 2008).
26. P. Rodriguez-Viciana et al., Cancer targets in the Ras pathway. Cold Spring Harb Symp Quant Biol 70, 461-467 (2005).
27. A. Toker, M. Yoeli-Lerner, Akt signaling and cancer: surviving but not moving on. Cancer Res 66, 3963-3966 (Apr 15, 2006).
28. V. D. Longo, M. R. Lieber, J. Vijg, Turning anti-ageing genes against cancer. Nat Rev Mol Cell Biol 9, 903-910 (Nov, 2008).
29. M. N. Pollak, E. S. Schernhammer, S. E. Hankinson, Insulin-like growth factors and neoplasia. Nat Rev Cancer 4, 505-518 (Jul, 2004).
30. A. G. Renehan et al., Insulin-like growth factor (IGF)-I, IGF binding protein-3, and cancer risk: systematic review and meta-regression analysis. Lancet 363, 1346-1353 (Apr 24, 2004).
31. F. P. Dominici, G. Arostegui Diaz, A. Bartke, J. J. Kopchick, D. Turyn, Compensatory alterations of insulin signal transduction in liver of growth hormone receptor knockout mice. J Endocrinol 166, 579-590 (Sep, 2000).
32. F. P. Dominici, S. Hauck, D. P. Argentino, A. Bartke, D. Turyn, Increased insulin sensitivity and upregulation of insulin receptor, insulin receptor substrate (IRS)-1 and IRS-2 in liver of Ames dwarf mice. J Endocrinol 173, 81-94 (Apr, 2002).
33. M. M. Masternak, J. A. Panici, M. S. Bonkowski, L. F. Hughes, A. Bartke, Insulin sensitivity as a key mediator of growth hormone actions on longevity. J Gerontol A Biol Sci Med Sci 64, 516-521 (May, 2009).
34. J. L. Liu et al., Disruption of growth hormone receptor gene causes diminished pancreatic islet size and increased insulin sensitivity in mice. Am J Physiol Endocrinol Metab 287, E405-413 (Sep, 2004).
35. P. V. Carroll et al., Growth hormone deficiency in adulthood and the effects of growth hormone replacement: a review. Growth Hormone Research Society Scientific Committee. J Clin Endocrinol Metab 83, 382-395 (Feb, 1998).
36. J. O. Johansson, J. Fowelin, K. Landin, I. Lager, B. A. Bengtsson, Growth hormone-deficient adults are insulin-resistant. Metabolism 44, 1126-1129 (Sep, 1995).
37. M. Bramnert et al., Growth hormone replacement therapy induces insulin resistance by activating the glucose-fatty acid cycle. J Clin Endocrinol Metab 88, 1455-1463 (Apr, 2003).
38. U.S., (Census Bureau, 2010).
39. J. Guevara-Aguirre, A. L. Rosenbloom, P. J. Fielder, F. B. Diamond, Jr., R. G. Rosenfeld, Growth hormone receptor deficiency in Ecuador: clinical and biochemical phenotype in two populations. J Clin Endocrinol Metab 76, 417-423 (Feb, 1993).
40. A. L. Rosenbloom, J. Guevara Aguirre, R. G. Rosenfeld, P. J. Fielder, The little women of Loja--growth hormone-receptor deficiency in an inbred population of southern Ecuador. N Engl J Med 323, 1367-1374 (Nov 15, 1990).
41. L. K. Bachrach et al., Bone mineral, histomorphometry, and body composition in adults with growth hormone receptor deficiency. J Bone Miner Res 13, 415-421 (Mar, 1998).
42. M. A. Berg, J. Guevara-Aguirre, A. L. Rosenbloom, R. G. Rosenfeld, U. Francke, Mutation creating a new splice site in the growth hormone receptor genes of 37 Ecuadorean patients with Laron syndrome. Hum Mutat 1, 24-32 (1992).
43. S. Amselem et al., Recurrent nonsense mutations in the growth hormone receptor from patients with Laron dwarfism. J Clin Invest 87, 1098-1102 (Mar, 1991).
44. M. A. Berg et al., Receptor mutations and haplotypes in growth hormone receptor deficiency: a global survey and identification of the Ecuadorean E180splice mutation in an oriental Jewish patient. Acta Paediatr Suppl 399, 112-114 (Apr, 1994).
45. A. L. Rosenbloom, J. Guevara-Aguirre, Growth hormone receptor deficiency in South America: colonial history, molecular biology, and growth and metabolic insights. J Pediatr Endocrinol Metab 21, 1107-1109 (Dec, 2008).
46. M. A. Berg et al., Diverse growth hormone receptor gene mutations in Laron syndrome. Am J Hum Genet 52, 998-1005 (May, 1993).
47. J. Guevara-Aguirre et al., Growth hormone receptor deficiency (Laron syndrome): clinical and genetic characteristics. Acta Paediatr Scand Suppl 377, 96-103 (1991).
48. *http:*//*www.who.int*/*en*/*.*
49. J. E. Shaw, R. A. Sicree, P. Z. Zimmet, Global estimates of the prevalence of diabetes for 2010 and 2030. Diabetes Res Clin Pract 87, 4-14 (Jan).
50. N. J. Hopwood, P. J. Forsman, F. M. Kenny, A. L. Drash, Hypoglycemia in hypopituitary children. Am J Dis Child 129, 918-926 (Aug, 1975).
51. M. W. Haymond, I. Karl, V. V. Weldon, A. S. Pagliara, The role of growth hormone and cortisone on glucose and gluconeogenic substrate regulation in fasted hypopituitary children. J Clin Endocrinol Metab 42, 846-856 (May, 1976).
52. Z. Laron, Y. Avitzur, B. Klinger, Carbohydrate metabolism in primary growth hormone resistance (Laron syndrome) before and during insulin-like growth factor-I treatment. Metabolism 44, 113-118 (Oct, 1995).
53. T. Rosen, B. A. Bengtsson, Premature mortality due to cardiovascular disease in hypopituitarism. Lancet 336, 285-288 (Aug 4, 1990).
54. D. R. Matthews et al., Homeostasis model assessment: insulin resistance and beta-cell function from fasting plasma glucose and insulin concentrations in man. Diabetologia 28, 412-419 (Jul, 1985).
55. *Materials and methods are available as supporting material on Science Online.*
56. M. H. Aguiar-Oliveira et al., Longevity in untreated congenital growth hormone deficiency due to a homozygous mutation in the GHRH receptor gene. J Clin Endocrinol Metab 95, 714-721 (Feb, 2010 ).
57. R. de Cabo et al., An in vitro model of caloric restriction. Exp Gerontol 38, 631-639 (Jun, 2003).
58. T. H. Ngo, R. J. Barnard, P. S. Leung, P. Cohen, W. J. Aronson, Insulin-like growth factor I (IGF-I) and IGF binding protein-1 modulate prostate cancer cell growth and apoptosis: possible mediators for the effects of diet and exercise on cancer cell survival. Endocrinology 144, 2319-2324 (Jun, 2003).
59. A. Csiszar et al., Anti-oxidative and anti-inflammatory vasoprotective effects of caloric restriction in aging: role of circulating factors and SIRT1. Mech Ageing Dev 130, 518-527 (Aug, 2009).
60. C. Sell et al., Simian virus 40 large tumor antigen is unable to transform mouse embryonic fibroblasts lacking type 1 insulin-like growth factor receptor. Proc Natl Acad Sci U S A 90, 11217-11221 (Dec 1, 1993).
61. G. Rena, S. Guo, S. C. Cichy, T. G. Unterman, P. Cohen, Phosphorylation of the transcription factor forkhead family member FKHR by protein kinase B. J Biol Chem 274, 17179-17183 (Jun 11, 1999).
62. A. Brunet et al., Akt promotes cell survival by phosphorylating and inhibiting a Forkhead transcription factor. Cell 96, 857-868 (Mar 19, 1999).
63. D. R. Alessi et al., Mechanism of activation of protein kinase B by insulin and IGF-1. EMBO J 15, 6541-6551 (Dec 2, 1996).
64. H. Huang et al., Skp2 inhibits FOXO1 in tumor suppression through ubiquitin-mediated degradation. Proc Natl Acad Sci U S A 102, 1649-1654 (Feb 1, 2005).
65. G. J. Kops et al., Forkhead transcription factor FOXO3a protects quiescent cells from oxidative stress. Nature 419, 316-321 (Sep 19, 2002).
66. P. F. Dijkers, R. H. Medema, J. W. Lammers, L. Koenderman, P. J. Coffer, Expression of the pro-apoptotic Bcl-2 family member Bim is regulated by the forkhead transcription factor FKHR-L1. Curr Biol 10, 1201-1204 (Oct 5, 2000).
67. S. Y. Kim, D. J. Volsky, PAGE: parametric analysis of gene set enrichment. BMC Bioinformatics 6, 144 (2005).
68. L. Fontana, L. Partridge, V. D. Longo, Extending healthy life span--from yeast to humans. Science 328, 321-326 (Apr 16).
69. L. Hlavata, T. Nystrom, Ras proteins control mitochondrial biogenesis and function in Saccharomyces cerevisiae. Folia Microbial (Praha) 48, 725-730 (2003).
70. J. Urban et al., Sch9 is a major target of TORC1 in Saccharomyces cerevisiae. Mol Cell 26, 663-674 (Jun 8, 2007).
71. R. A. Busuttil et al., Organ-specific increase in mutation accumulation and apoptosis rate in CuZn-superoxide dismutase-deficient mice. Cancer Res 65, 11271-11275 (Dec 15, 2005).
72. C. Chen, K. Umezu, R. D. Kolodner, Chromosomal rearrangements occur in S. cerevisiae rfa1 mutator mutants due to mutagenic lesions processed by double-strand-break repair. Mol Cell 2, 9-22 (Jul, 1998).
73. O. Shevah, Z. Laron, Patients with congenital deficiency of IGF-I seem protected from the development of malignancies: a preliminary report. Growth Horm IGF Res 17, 54-57 (Feb, 2007).
74. M. H. Aguiar-Oliveira et al., Longevity in untreated congenital growth hormone deficiency due to a homozygous mutation in the GHRH receptor gene. J Clin Endocrinol Metab 95, 714-721 (Feb).
75. Y. Suh et al., Functionally significant insulin-like growth factor I receptor mutations in centenarians. Proc Natl Acad Sci U S A 105, 3438-3442 (Mar 4, 2008).
76. M. Parrizas, D. LeRoith, Insulin-like growth factor-1 inhibition of apoptosis is associated with increased expression of the bcl-xL gene product. Endocrinology 138, 1355-1358 (Mar, 1997).
77. M. A. Kennedy, S. G. Rakoczy, H. M. Brown-Borg, Long-living Ames dwarf mouse hepatocytes readily undergo apoptosis. Exp Gerontol 38, 997-1008 (Sep, 2003).
78. V. D. Longo, The Ras and Sch9 pathways regulate stress resistance and longevity. Exp Gerontol 38, 807-811 (Jul, 2003).
79. L. Hlavata, L. Nachin, P. Jezek, T. Nystrom, Elevated Ras/protein kinase A activity in Saccharomyces cerevisiae reduces proliferation rate and lifespan by two different reactive oxygen species-dependent routes. Aging Cell 7, 148-157 (Mar, 2008).
80. Y. Li, W. Xu, M. W. McBurney, V. D. Longo, SirT1 inhibition reduces IGF-I/IRS-2/Ras/ERK1/2 signaling and protects neurons. Cell Metab 8, 38-48 (Jul, 2008).
81. P. Fabrizio et al., SOD2 functions downstream of Sch9 to extend longevity in yeast. Genetics 163, 35-46 (Jan, 2003).
82. D. l. T. W. Guevara-Aguirre J, Rosenbloom AL, Acosta M, Rosenfeld RG, paper presented at the 73rd Annual Meeting of the Endocrine Society, Washington, DC, Bethesda, MD, 1991.
83. A. Bartke, Minireview: role of the growth hormone/insulin-like growth factor system in mammalian aging. Endocrinology 146, 3718-3723 (Sep, 2005).
84. M. Bluher, B. B. Kahn, C. R. Kahn, Extended longevity in mice lacking the insulin receptor in adipose tissue. Science 299, 572-574 (Jan 24, 2003).
85. P. L. Olive, J. P. Banath, The comet assay: a method to measure DNA damage in individual cells. Nat Protoc 1, 23-29 (2006).
86. C. Cheadle, M. P. Vawter, W. J. Freed, K. G. Becker, Analysis of microarray data using Z score transformation. J Mol Diagn 5, 73-81 (May, 2003).
87. C. B. Brachmann et al., Designer deletion strains derived from Saccharomyces cerevisiae S288C: a useful set of strains and plasmids for PCR-mediated gene disruption and other applications. Yeast 14, 115-132 (Jan 30, 1998).
88. C. Kaiser, M. S., M. A., Methods in yeast genetics. (Cold Spring Harbor Laboratory Press, New York, ed. 1994, 1994), vol. 234.
89. P. Fabrizio, V. D. Longo, The chronological life span of Saccharomyces cerevisiae. Aging Cell 2, 73-81 (Apr, 2003).
90. F. Madia, C. Gattazzo, P. Fabrizio, V. D. Longo, A simple model system for age-dependent DNA damage and cancer. Mech Ageing Dev 128, 45-49 (Jan, 2007).

## Claims

1. A GH/IGF-1 Axis inhibitory composition for use in a method of alleviating a side effect of chemotherapy in a subject, the method comprising:
identifying a subject undergoing chemotherapy;
administering a therapeutically effective amount of a GH/IGF-1 Axis inhibitory composition to the subject, and measuring the level of IGF-1 in the subject;
wherein the GH/IGF-1 Axis inhibitory composition comprises a human growth hormone variant that includes at least one amino acid substitution selected from the group consisting of H18D, H21N, R167N, K168A, D171S, K172R, E174S, I179T, and G120R or a recombinant 191 amino acid analog of the GH protein which has appended polyethyleneglycol groups.

2. The GH/IGF-1 Axis inhibitory composition for use of claim 1 wherein the human growth hormone variant includes the amino acid substitutions H18D, H21N, R167N, K168A, D171S, K172R, E174S, I179T, and G120R.

3. The composition for use of claim 1 or claim 2 wherein the side effect of chemotherapy that is alleviated being selected from the group consisting of weight loss, hair loss, gastrointestinal disturbances, alteration of blood chemistry and composition, and immune suppression.

4. A kit for use in a method of alleviating a side effect of chemotherapy in a subject, the kit comprising:
one or more doses of a GH/IGF-1 Axis inhibitory composition, the GH/IGF-1 Axis inhibitory composition comprising a human growth hormone variant that includes at least one amino acid substitution selected from the group consisting of H18D, H21N, R167N, K168A, D171S, K172R, E174S, I179T, and G120R or a recombinant 191 amino acid analog of the GH protein which has appended polyethyleneglycol groups.;
instructions indicating that the a GH/IGF-1 Axis inhibitory composition is to be provided to a subject undergoing chemotherapy; and
a vessel for holding a blood sample drawn from the subject to be used to monitor levels of IGF-1 and/or insulin in the case of diabetes.

5. The kit of claim 4 wherein the human growth hormone variant includes the amino acid substitutions H18D, H21N, R167N, K168A, D171S, K172R, E174S, I179T, and G120R

6. The kit of claim 4 or claim 5 wherein the side effect of chemotherapy that is alleviated being selected from the group consisting of weight loss, hair loss, gastrointestinal disturbances, alteration of blood chemistry and composition, and immune suppression.

## Patentansprüche

1. Eine die GH/IGF-1-Achse inhibierende Zusammensetzung zur Anwendung in einem Verfahren zur Linderung einer Nebenwirkung der Chemotherapie in einem Subjekt, wobei das Verfahren umfasst:
das Identifizieren eines Subjekts, an dem eine Chemotherapie durchgeführt wird;
das Verabreichen einer therapeutisch wirksamen Menge einer die GH/IGF-1-Achse inhibierenden Zusammensetzung an das Subjekt; und
das Messen des IGF-1-Spiegels in dem Subjekt;
wobei die die GH/IGF-1-Achse inhibierende Zusammensetzung eine Variante eines humanen Wachstumshormons, die mindestens eine Aminosäuresubstitution einschließt, die ausgewählt ist aus der Gruppe bestehend aus H18D, H21N, R167N, K168A, D171S, K172R, E174S, I179T und G120R, oder ein rekombinantes 191-Aminosäuren-Analogon des GH-Proteins mit angehängten Polyethylenglykolgruppen umfasst.

2. Die GH/IGF-1-Achse inhibierende Zusammensetzung zur Anwendung nach Anspruch 1, wobei die Variante eines humanen Wachstumshormons die Aminosäuresubstitutionen H18D, H21N, R167N, K168A, D171S, K172R, E174S, I179T und G120R einschließt.

3. Zusammensetzung zur Anwendung nach Anspruch 1 oder Anspruch 2, wobei die Nebenwirkung der Chemotherapie, die gelindert wird, ausgewählt ist aus der Gruppe bestehend aus Gewichtsverlust, Haarausfall, gastrointestinalen Störungen, Veränderung der Chemie und Zusammensetzung des Blutes, sowie Immunsuppression.

4. Kit zur Anwendung in einem Verfahren zur Linderung einer Nebenwirkung der Chemotherapie in einem Subjekt, wobei das Kit umfasst:
eine oder mehrere Dosen einer die GH/IGF-1-Achse inhibierenden Zusammensetzung, die die GH/IGF-1-Achse inhibierende Zusammensetzung umfassend eine Variante eines humanen Wachstumshormons, die mindestens eine Aminosäuresubstitution einschließt, die ausgewählt ist aus der Gruppe bestehend aus H18D, H21N, R167N, K168A, D171S, K172R, E174S, I179T und G120R, oder ein rekombinantes 191-Aminosäure-Analogon des GH-Proteins mit angehängten Polyethylenglykolgruppen;
eine Anleitung, die angibt, dass die die GH/IGF-1-Achse inhibierende Zusammensetzung einem Subjekt bereitzustellen ist, an dem eine Chemotherapie durchgeführt wird; und
ein Gefäß zur Aufnahme einer Blutprobe, die von dem Subjekt erhalten wurde und zur Verfolgung der Spiegel von IGF-1 und/oder Insulin (im Fall von Diabetes) zu verwenden ist.

5. Kit nach Anspruch 4, wobei die Variante des humanen Wachstumshormons die Aminosäuresubstitutionen H18D, H21N, R167N, K168A, D171S, K172R, E174S, I179T und G120R einschließt.

6. Kit nach Anspruch 4 oder Anspruch 5, wobei die Nebenwirkung der Chemotherapie, die gelindert wird, ausgewählt ist aus der Gruppe bestehend aus Gewichtsverlust, Haarausfall, gastrointestinalen Störungen, Veränderung der Chemie und Zusammensetzung des Blutes, sowie Immunsuppression.

## Revendications

1. Composition inhibitrice de l'axe GH/IGF-1 pour une utilisation dans une méthode de soulagement d'un effet secondaire de chimiothérapie chez un sujet, le procédé comprenant :
identifier un sujet subissant une chimiothérapie ;
administrer au sujet une quantité thérapeutiquement efficace d'une composition inhibitrice de l'axe GH/IGF-1, et
mesurer le taux d'IGF-1 chez le sujet ;
la composition inhibitrice de l'axe GH/IGF-1 comprenant un variant d'hormone de croissance humaine qui comprend au moins une substitution d'acide aminé choisie dans le groupe consistant en H18D, H21N, R167N, K168A, D171S, K172R, E174S, I179T et G120R ou un analogue de 191 acides aminés recombinant de la protéine GH qui a des groupes de polyéthylèneglycol pendants.

2. Composition inhibitrice de l'axe GH/IGF-1 pour une utilisation selon la revendication 1, dans laquelle le variant d'hormone de croissance humaine comprend les substitutions d'acides aminés H18D, H21N, R167N, K168A, D171S, K172R, E174S, I179T et G120R.

3. Composition pour une utilisation selon l'une des revendications 1 ou 2, dans laquelle l'effet secondaire de chimiothérapie qui est soulagé est choisi dans le groupe consistant en la perte de poids, la perte de cheveux, les perturbations gastro-intestinales, la modification de la chimie et de la composition du sang et l'immunosuppression.

4. Coffret pour une utilisation dans une méthode de soulagement d'un effet secondaire de chimiothérapie chez un sujet, le coffret comprenant :
une ou plusieurs doses d'une composition inhibitrice de l'axe GH/IGF-1, la composition inhibitrice de l'axe GH/IGF-1 comprenant un variant d'hormone de croissance humaine qui comprend au moins une substitution d'acide aminé choisie dans le groupe consistant en H18D, H21N, R167N, K168A, D171S, K172R, E174S, I179T et G120R ou un analogue de 191 acides aminés recombinant de la protéine GH qui a des groupes polyéthylèneglycol pendants ;
des instructions indiquant que la composition inhibitrice de l'axe GH/IGF-1 doit être fournie à un sujet subissant une chimiothérapie ; et
un récipient pour contenir un échantillon de sang prélevé à partir du sujet devant être utilisé pour surveiller les taux d'IGF-1 et/ou d'insuline dans le cas du diabète.

5. Coffret selon la revendication 4, dans lequel le variant d'hormone de croissance humaine comprend les substitutions d'acides aminés H18D, H21N, R167N, K168A, D171S, K172R, E174S, I179T et G120R.

6. Coffret selon l'une des revendications 4 ou 5, dans lequel l'effet secondaire de la chimiothérapie qui est soulagé est choisi dans le groupe consistant en la perte de poids, la perte de cheveux, les perturbations gastro-intestinales, la modification de la chimie et de la composition du sang et l'immunosuppression.
